(19) Europäisches Patentamt
European Patent Office
Office européen des brevets

(11) Publication number: **0 114 750**
**A2**

# (12) EUROPEAN PATENT APPLICATION

(21) Application number: **84300336.9**

(22) Date of filing: **19.01.84**

(51) Int. Cl.[3]: **C 07 D 501/57**
**A 61 K 31/575**

(30) Priority: **24.01.83 GB 8301874**
**24.01.83 GB 8301876**
**21.07.83 GB 8319705**
**26.08.83 GB 8322952**

(43) Date of publication of application:
**01.08.84 Bulletin 84/31**

(84) Designated Contracting States:
**BE CH DE FR GB IT LI NL SE**

(71) Applicant: **BEECHAM GROUP PLC**
**Beecham House Great West Road**
**Brentford Middlesex(GB)**

(72) Inventor: **Milner, Peter Henry**
**20 Goose Green Close**
**Horsham Sussex(GB)**

(74) Representative: **Hesketh, Alan, Dr. et al,**
**European Patent Attorney Beecham Pharmaceuticals**
**Great Burgh Yew Tree Bottom Road**
**Epsom, Surrey, KT18 5XQ(GB)**

## (54) Beta-lactam antibacterial agents.

(57) A compound of formula (I) or a pharmaceutically acceptable salt or *in vivo* hydrolysable ester thereof:

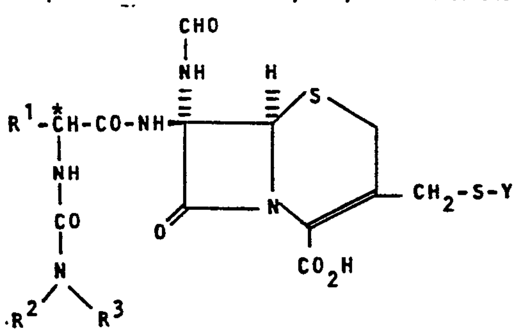

(I)

wherein Y is a group of formula (a), (b), (c), or (d):

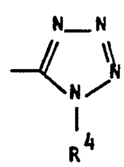

(a)

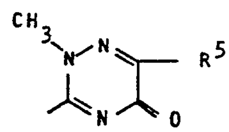

(c)

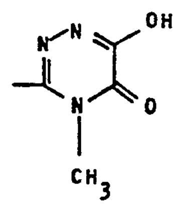

(b)

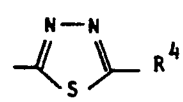

(d)

./...

**EP 0 114 750 A2**

$R^1$ is phenyl, substituted phenyl, cyclohexenyl, cyclohexadienyl, or a 5- or 6-membered heterocyclic ring containing up to three hetero-atoms selected from oxygen, sulphur or nitrogen, optionally substituted with hydroxy, amino, halogen, substituted amino or $C_{1-6}$ alkoxy; $R^2$ is hydrogen or an optionally substituted $C_{1-6}$ alkyl group and $R^3$ is an optionally substituted 5- or 6- membered heterocyclic group containing one or two nitrogen heteroatoms; or $R^2$ and $R^3$ together with the nitrogen atom to which they are attached form an optionally substituted five- or six-membered heterocyclic group containing one or two nitrogen heteroatoms; $R^4$ is carbamoyl, carbamoyl ($C_{1-6}$) alkyl, amino ($C_{1-6}$) alkyl, di ($C_{1-6}$ alkyl) amino ($C_{1-6}$) alkyl, hydroxy ($C_{1-6}$) alkyl, sulpho ($C_{1-6}$) alkyl, $C_{2-6}$ alkenyl or carboxy ($C_{1-6}$) alkyl; and $R^5$ is carbamoyl or $C_{1-6}$ alkyl.

# β-LACTAM ANTIBACTERIAL AGENTS

This invention relates to a class of novel β-lactam derivatives, which have antibacterial activity and are of value in the treatment of infections in animals especially mammals including man caused by a wide range of organisms, particularly Gram-negative organisms. The invention also relates to a process for the preparation of such compounds, intermediates for use in the preparation of the compounds and to pharmaceutical compositions comprising the antibacterially active compounds.

The present invention provides a compound of formula (I) or a pharmaceutically acceptable salt or <u>in vivo</u> hydrolysable ester thereof:

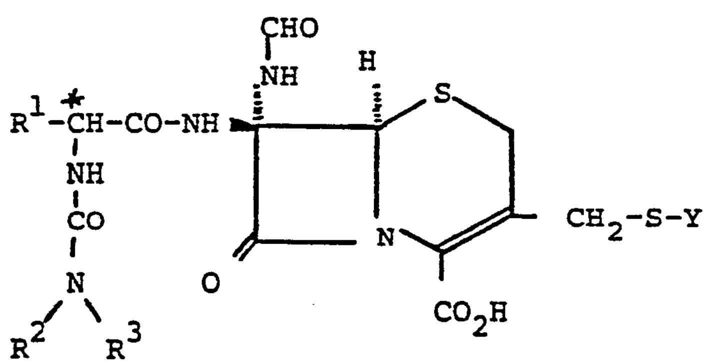

(I)

wherein Y is a group of formula (a), (b), (c), or (d):

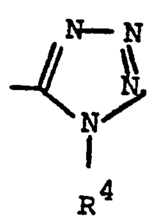

(a)

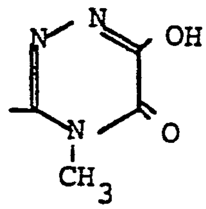

(b)

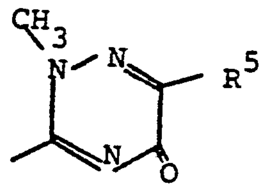

(c)

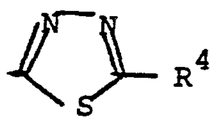

(d)

$R^1$ is phenyl, substituted phenyl, cyclohexenyl, cyclohexadienyl, or a 5- or 6-membered heterocyclic ring containing up to three hetero-atoms selected from oxygen, sulphur or nitrogen, optionally substituted with hydroxy, amino, halogen, substituted amino or $C_{1-6}$ alkoxy; $R^2$ is hydrogen or an optionally substituted $C_{1-6}$ alkyl group and $R^3$ is an optionally substituted 5- or 6- membered heterocyclic group containing one or two nitrogen heteroatoms; or $R^2$ and $R^3$ together with the nitrogen atom to which they are attached form an optionally substituted five- or six-membered heterocyclic group containing one or two nitrogen heteroatoms;

$R^4$ is carbamoyl, carbamoyl ($C_{1-6}$) alkyl, amino ($C_{1-6}$) alkyl, di ($C_{1-6}$ alkyl) amino ($C_{1-6}$) alkyl, hydroxy ($C_{1-6}$) alkyl, sulpho ($C_{1-6}$) alkyl, $C_{2-6}$ alkenyl or carboxy ($C_{1-6}$) alkyl; and $R^5$ is carbamoyl or $C_{1-6}$ alkyl.

Suitably the substituted phenyl group for $R^1$ is a phenyl group substituted with up to three groups

selected from $C_{1-6}$ alkyl, phenyl, halogen, C1-6 alkoxy, amino, nitro, hydroxy, $C_{1-6}$ alkylamido, $C_{1-6}$ alkylcarbonyloxy, carboxy, $C_{1-6}$ alkoxycarbonyl, $C_{1-6}$ alkoxycarbonyloxy, halo ($C_{1-6}$) alkyl, oxo ($C_{1-6}$) alkyl, $C_{1-6}$ alkylcarbonyl, aryloxy, aralkyloxy, arylcarbonyl, $C_{1-6}$ alkylamino or di($C_{1-6}$) alkylamino.

In formula (I), the group $R^1$ is preferably phenyl, 4-hydroxyphenyl, 3,4-di($C_{1-6}$ alkylcarbonyloxy)-phenyl, 3,4-dihydroxyphenyl, 2-thienyl, 3-thienyl or 2-amino-4-thiazolyl.

Particularly preferred groups $R^1$ are phenyl, 3,4-dihydroxyphenyl and 3,4-diacetoxyphenyl.

Suitably $R^2$ is hydrogen or $C_{1-6}$ alkyl.

More suitably $R^2$ is hydrogen.

Suitable substituents for the 5- or 6- membered heterocyclic group of $R^3$ or $R^2$ and $R^3$ together include the optionally substituted alkyl, alkenyl, alkynyl, cycloalkyl or cycloalkenyl group; optionally substituted phenyl, oxo; the hydroxy group optionally substituted by alkyl, alkenyl, cycloalkyl, phenyl, pyridyl, pyrimidyl or benzyl; the optionally substituted mercapto group, the alkylsulphonyl group; the substituted imino group; or the amino group optionally substituted by an alkyl, alkenyl, cycloalkyl, phenyl, substituted phenyl or benzyl group. Alternatively two substituents on the ring may form the residue of a further carbocyclic or heterocyclic ring.

Suitably Y is a group of formula (a) or (b).
Suitably Y is a group of formula (a) or (d).
More suitably Y is:

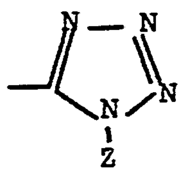

or

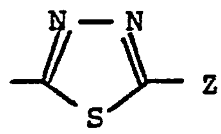

wherein Z is carbamoyl, carbamoyl ($C_{1-6}$) alkyl, amino ($C_{1-6}$) alkyl, di- ($C_{1-6}$ alkyl) amino ($C_{1-6}$) alkyl, hydroxy ($C_{1-6}$) alkyl, sulpho ($C_{1-6}$) alkyl, or $C_{2-6}$ alkenyl.

Suitably Z represents carbamoyl, carbamoylmethyl, aminomethyl, aminoethyl, dimethyl aminomethyl, dimethylaminoethyl, hydroxymethyl, hydroxyethyl, sulphomethyl and ethenyl.

Further suitable values of Y include:

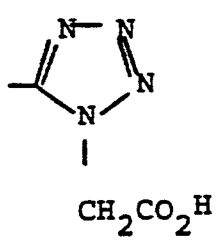

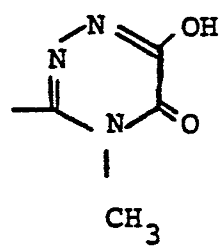

The carbon atom marked * in formulae herein is asymmetric so that the compounds may exist as two optically active diastereoisomers. In general that prepared from the D-side chain exhibits the highest

antibacterial activity and accordingly the D compound or the DL mixtures are preferred, with the D compound being particularly preferred.

The compounds of the present invention may contain both an amino group and/or a carboxyl group and may, therefore, exist as the zwitterion or may form salts with suitable acids or bases.

The formamido group can exist in two preferred conformations, those wherein the hydrogen atoms of the -NH-CHO are, *cis*- or *trans*-, of which the *cis* conformation normally predominates.

Since the β-lactam antibiotic compounds of the present invention are intended for use in pharmaceutical compositions it will readily be understood that they are each provided in substantially pure form, for example at least 50% pure, more suitably at least 75% pure and preferably at least 95% pure (% are on a wt/wt basis). Impure preparations of the compounds may be used for preparing the more pure forms used in the pharmaceutical compositions. Although the purity of intermediate compounds of the present invention is less critical it will readily be understood that the substantially pure form is preferred as for the β-lactam antibiotic compounds. Preferably, whenever possible, the compounds of the present invention are obtained in crystalline form.

Examples of suitable pharmaceutically acceptable *in vivo* hydrolysable ester groups include those which

break down readily in the human body to leave the parent acid or its salt. Suitable ester groups of this type include those of part formula (i), (ii) and (iii):

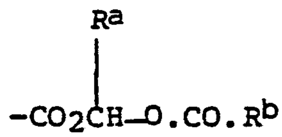

(i)

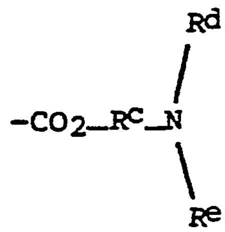

(ii)

$-CO_2CH_2-OR^f$ (iii)

wherein $R^a$ is hydrogen, methyl, or phenyl, $R^b$ is $C_{1-6}$ alkyl, $C_{1-6}$ alkoxy or phenyl; or $R^a$ and $R^b$ together form a 1,2-phenylene group optionally substituted by one or two methoxy groups; $R^c$ represents $C_{1-6}$ alkylene optionally substituted with a methyl or ethyl group - $R^d$ and $R^e$ independently represent $C_{1-6}$ alkyl; $R^f$ represents $C_{1-6}$ alkyl. Examples of suitable <u>in vivo</u> hydrolysable ester group include for example acyloxyalkyl groups such as acetoxymethyl, pivaloyloxymethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a

β-lactamase inhibitor.

Suitable pharmaceutically acceptable salts of the carboxy group of the compound of formula (I) include metal salts eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylenediamine, 1-ephenamine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins. Other suitable salts include the lithium and silver salt.

Some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation.

One particularly preferred sub-group within the present invention provides a compound of formula (II) or a pharmaceutically acceptable salt or *in vivo*

hydrolysable ester thereof:

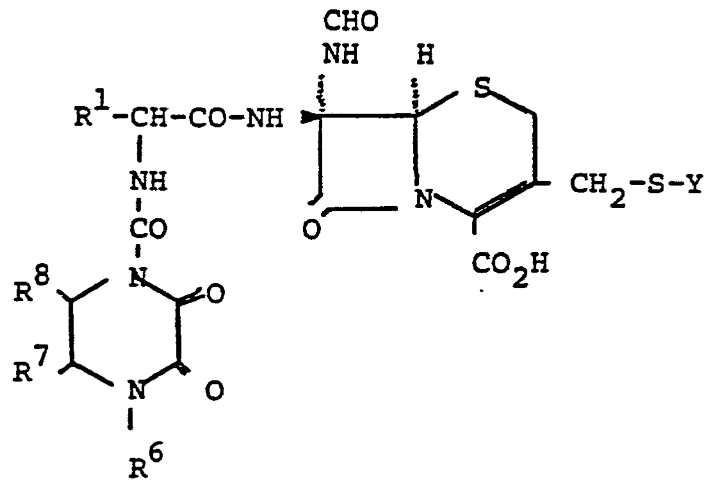

(II)

wherein $R^1$ and Y are as defined with respect to formula (I) and $R^6$ represents hydrogen, $C_{1-6}$ alkyl, substituted alkyl, aryl, or aralkyl; $R^7$ and $R^8$ are the same or different and represent hydrogen, $C_{1-6}$ alkyl, substituted alkyl, halogen, amino, hydroxy or $C_{1-6}$ alkoxy or $R^7$ and $R^8$ form the residue of 5- or 6-membered carbocyclic or heterocyclic ring.

Suitable $C_{1-6}$ alkyl groups for the groups $R^6$, $R^7$ and $R^8$ in formula (II) include methyl, ethyl, n- and iso-propyl, n, sec-, iso- and tert-butyl. Preferably $R^6$ is ethyl. Preferably $R^7$ and $R^8$ are hydrogen.

Specific compounds within this invention include the following and pharmaceutically acceptable salts and *in-vivo* hydrolysable esters thereof:

a) 7β-[DL-2-(3,4-Diacetoxyphenyl)-2[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid;

3-[(1-Carboxymethyl-1H-tetrazol-5-yl)thiomethyl]-7β-[D-2-2-[(4-ethyl-2,3-dioxopiperazin-1-yl)carbonylamino]-2-phenylacetamido]-7α-formamidoceph-3-em-4-carboxylic acid; and

b) 7β[D-2-[(4-Ethyl-2,3-dioxopiperazin-1-yl)carbonylamino]-2-phenylacetamido]-7α-formamido-3-[(6-hydroxy-4-methyl-5-oxo-4H-1,2,4-triazin-3-yl)thiomethyl]ceph-3-em-4-carboxylic acid;

7β-[D-2(4-Acetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid;

7β-[DL-2-[(4-Ethyl-2,3-dioxopiperazin-1-yl)carbonylamino]-2-(thien-2-yl)acetamido]-7α-formamido-3-[(6-hydroxy-4-methyl-5-oxo-4H-1,2,4-triazin-3-yl)thiomethyl]ceph-3-em-4-carboxylic acid;

7β-[DL-2[(4-Ethyl-2,3-dioxopiperazin-1-yl)carbonylamino]-2-(2-fluorophenyl)acetamido]-7α-formamido-3-[(6-hydroxy-4-methyl-5-oxo-4H-1,2,4-triazin-3-yl)thiomethyl]ceph-3-em-4-carboxylic acid;

7β-[D-2(3,4-Diacetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid, disodium;

3-[(1-Carboxymethyl-1H-tetrazol-5-yl)thiomethyl]-7β-[D-2(3,4-diacetoxyphenyl)-2-[(4-ethyl-2,3-dioxopiperazin-1-yl)carbonylamino]acetamido]-7α-formamidoceph-3-em-4-carboxylic acid; and

c) 3-[(1-carbamoylmethyl-1H-tetrazol-5-yl)thiomethyl]-7α-[D-2-[(4-ethyl-2,3-dioxopiperazin-1-yl)-carbonylamino]-2-pheynylacetamido]-7α-formamidoceph-3-em-4-carboxylic acid; and

d) 3-[(1-Carbamoylmethyl-1H-tetrazol-5-yl)thiomethyl]-7β-[D-2-(3,4-diacetoxyphenyl)-2-[(4-ethyl-2,3-dioxopiperazin-1-yl)carbonylamino]-7α-formamidoceph-3-em-4-carboxylic acid; and

e) 3-[(1-Carboxymethyl-1H-tetrazol-5-yl)thiomethyl]-7β-[D-2-(3,4-dihydroxyphenyl)-2-[(4-ethyl-2,3-dioxopiperazin-1-yl)carbonylamino]acetamido]-7α-formamidoceph-3-em-4-carboxylic acid;

3-[(1-Dimethylaminoethyl-1H-tetrazol-5-yl)thiomethyl]-7β-D-2(4-ethyl-2,3-dioxopiperaxin-1-yl)carbonylamino]-2-phenylacetamido]-7α-formamidoceph-3-em-4-carboxylic acid; and

f) 3-[(6-Carbamoyl-2-methyl-5-oxo-4H-1,2,4-triazin-3-yl)thomethyl]-7β-[D-2-[(4-ethyl-2,3-dioxopiperazin-1-yl)carbonylamino]-2-phenylacetamido]-7α-formamidoceph-3-em-4-carboxylic acid.

The antibiotic compounds according to the invention may be formulated for administration in any convenient way for use in human or veterinary medicine, according to techniques and procedures *per se* known in the art with reference to other antibiotics, and the invention therefore includes within its scope a pharmaceutical composition comprising an antibiotic compound according to the present invention such as, for example a compound of formula (I) above together with a pharmaceutically acceptable carrier or excipient.

The compositions may be formulated for administration by any suitable route, such as oral or parenteral, or by topical application. The compositions may be in the form of tablets, capsules, powders, granules, lozenges, creams or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The

tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulsoe, carboxymethyl cellulose, aluminium stearate gel or hydrogenated edible fats, emulsifying agents, for example lecithin, sorbitan monooleaate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and if desired conventional flavouring or colouring agents.

Suppositories will contain conventional suppository base, eg cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as local anaesthetic, preservative and buffering agents can be dissolved in

the vehicle. To enhance the stability, the composition can be frozen after filling into the vial and the water removed under vacuum. The dry lyophilised powder is the sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 10000 mg per day, for instance 1500 mg per day depending on the route and frequency of administration. Such a dosage corresponds to 1.5 to 50 mg/kg per may. Suitably the dosage is from 5 to 20 mg/kg per day.

The antibiotic compounds according to the present invention may be the sole therapeutic agent in the compositions of the invention or a combination with other antibiotics and/or β-lactamase inhibitor may be

employed.

Advantageously the compositions also comprise a compound of formula (III) or a pharmaceutically acceptable salt or ester thereof:

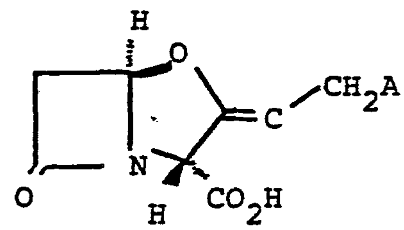

(III)

wherein A is hydroxyl, substituted hydroxyl, thiol, substituted thiol, amino, mono- or di-hydrocarbyl substituted amino, or mono- or di-acylamino.

A further advantageous composition comprises an antibiotic compound according to the invention together with a β-lactamase inhibitor of formula (IV) or a pharmaceutically acceptable salt or *in vivo* hydrolysable ester thereof:

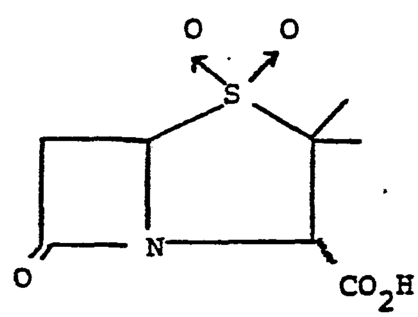

(IV)

and a pharmaceutically acceptable carrier or excipient.

Further suitable β-lactamase inhibitors include 6β-bromopenicillanic acid and salts and *in vivo* hydrolysable esters and 6β-iodopenicillanic acid and salts and *in vivo* hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention.

The antibiotic compounds of the present invention are active against a broad range of gram positive and gram negative bacteria, in particular they are useful for treatment of respiratory tract and urinary tract infections in humans and mastitis in cattle. A particular advantage of the antibacterially active compounds of this invention is their stability to β-lactamase enzymes and they are therefore effective against β-lactamase producing organisms.

The present invention further provides a process for the preparation of a compound of formula (I) which process comprises formylating a compound of formula (V):

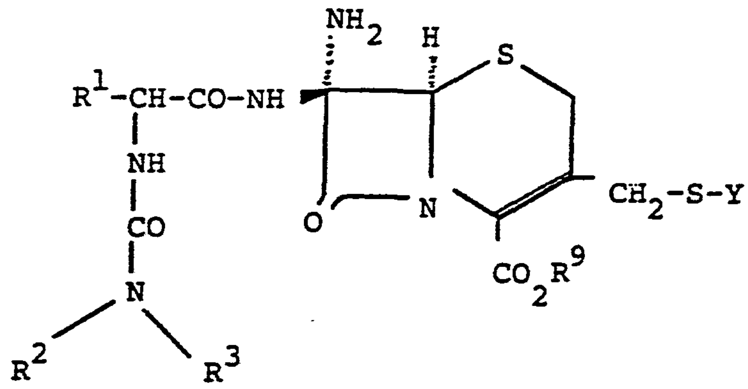

(V)

wherein $R^1$, $R^2$, $R^3$, and Y are as hereinbefore defined and wherein any reactive groups may be protected; and $R^9$ is a readily removable carboxy protecting group, and thereafter, if necessary, carrying out one or more of the following steps:

i) removing any carboxy protecting group $R^9$;

ii) removing any protecting groups on $R^1$, $R^2$, $R^3$ or Y;

iii) converting the product to a salt or <u>in vivo</u> hydrolysable ester.

Suitable formylating agents include mixed anhydrides such as formic acetic anhydride. The reaction may suitably be carried out in a temperature in the range -50°C to 30°C in aprotic solvent such as, for example, dichloromethane, chloroform, dimethylformamide, tetrahydrofuran, hexamethylphosphoramide, or dimethylsulphoxide, in the presence of a tertiary base. A preferred tertiary base employed in the

reaction is a base of the pyridine type, such as pyridine, lutidine or picoline.

Suitable readily removable carboxyl protecting groups for the group $-CO_2R^9$ in formula (V) include salt and ester derivatives of the carboxylic acid. The derivative is preferably one which may readily be cleaved.

Suitable ester-forming carboxyl-protecting groups are those which may be removed under conventional conditions. Such groups for $R^9$ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl, methoxymethyl, a silyl, stannyl or phosphorus-containing group, an oxime radical of formula $-N=CHR^o$ where $R^o$ is aryl or heterocyclic, or an in vivo hydrolysable ester radical such as defined above.

A carboxyl group may be regenerated from any of the above esters by usual methods appropriate to the particular $R^9$ group, for example, acid- and base-catalysed hydrolysis, or by enzymically -catalysed hydrolysis, or by hydrogenolysis.

A process for preparing compounds within formula (V) is

disclosed in or analogous to processes disclosed in US Patent No. 3,962,214 in UK Patent No. 1348984, and in European patent application No. 82303821.1.

The compounds of formula (I) may also suitably be prepared by reacting a compound of formula (VI):

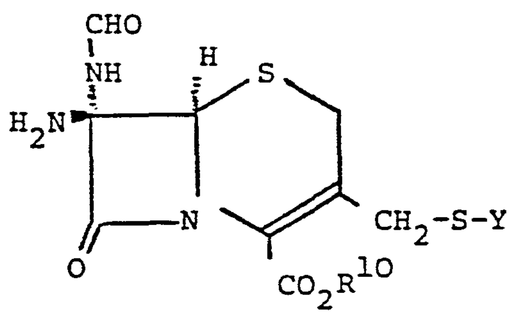

(VI)

wherein Y is as hereinbefore defined and wherein any reactive groups may be protected; the amino group is optionally substituted with a group which permits acylation to take place and $R^{10}$ is hydrogen or a group $R^9$ as hereinbefore defined; with an N-acylating derivative of an acid of formula (VII):

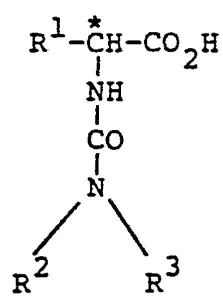

(VII)

wherein $R^1$, $R^2$ and $R^3$ are as defined with respect to formula (I) and wherein any reactive groups therein may be protected; and thereafter, if necessary, carrying out one or more of the following steps:

i) removing any carboxyl-protecting group $R^{10}$;
ii) removing any protecting groups on the $R^1$, $R^2$, $R^3$ or Y;
iii) converting the product into a salt or <u>in vivo</u> hydrolysable ester thereof.

Suitable groups which permit acylation to take place and which are optionally present on the amino group of the starting material of the formula (VI) include <u>N</u>-silyl, <u>N</u>-stannyl and <u>N</u>-phosphorus groups, for example trialkylsilyl groups such as trimethylsilyl, trialkyltin groups such as tri-n-butyltin, groups of formula $-P.R^aR^b$ wherein $R^a$ is an alkyl, haloalkyl, aryl, aralkyl, alkoxy, haloalkyl, aryl, aralkyl, alkoxy, haloalkoxy, aryloxy, aralkyloxy or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

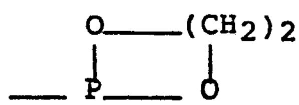
and
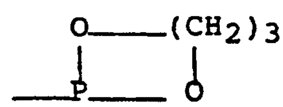

A reactive <u>N</u>-acylating derivative of the acid (VII) is

employed in the above process. The choice of reactive derivative will of course be influenced by the chemical nature of the substituents of the acid.

Suitable N-acylating derivatives include an acid halide, preferably the acid chloride or bromide. Acylation with an acid halide may be effected in the presence of an acid binding agent for example, tertiary amine (such as triethylamine, pyridine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide - such as ethylene oxide or propylene oxide. The acylation reaction using an acid halide may be carried out at a temperature in the range -50°C to +50°C, preferably -20°C to +20°c, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (VII) or a salt thereof with a halogenating (eg chlorinating or brominating) agent such as phosphorus pentachloride, thionyl chloride or oxalyl chloride.

Alternatively, the N-acylating derivative of the acid (VII) may be a symmetrical or mixed anhydride.

Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric or phosphorous acids) or aliphatic or aromatic sulphonic acids (such as p-toluenesulphonic acid). When a symmetrical anhydride is employed, the reaction may be carried out in the presence of 2,6-lutidine as catalyst.

Alternative N-acylating derivatives of acid (VII) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, or 8-hydroxyquinoline; or amides such as N-acylsaccharins, N-acylthiazolidin-2-thione or N-acylphthalimides; or an alkylidene iminoester prepared by reaction of the acid (VII) with an oxime.

Other reactive N-acylating derivatives of the acid (VII) include the reactive intermediates formed by reaction in situ with a condensing agent such as a carbodiimide, for example, N,N'-diethyl-, dipropyl- or diisopropylcarbodiimide, N,N'-di-cyclohexyl-carbodiimide, or N-ethyl-N'-[3-(dimethylamino)propyl]-carbodiimide; a suitable carbonyl compound, for example, N,N'-carbonyldiimidazole or N,N'-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl

0114750

2-ethoxy-1,2-dihydroquinoline. Other condensing agents include Lewis acids (for example $BBr_3 - C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

The compounds of formula (I) may also suitably be prepared by reacting a compound of formula (VIII):

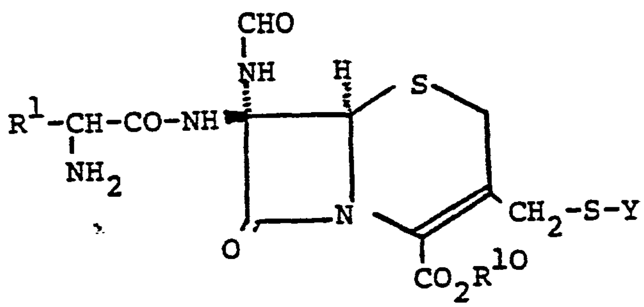

(VIII)

wherein $R^1$, $R^{10}$ and Y are as hereinbefore defined and the α-amino group is optionally substituted with a group which permits acylation to take place, and any reactive groups may be protected; with an N-acylating derivative of an acid of formula (IX):

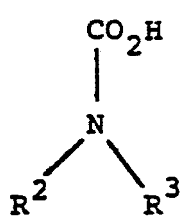

(IX)

wherein $R^2$ and $R^3$ are as hereinbefore defined and wherein any reactive groups may be protected; and thereafter, if necessary, carrying out one or more of the following steps:

i) removing any carboxyl-protecting group $R^{10}$;
ii) removing any protecting groups on $R^1$, $R^2$, $R^3$ or Y;
iii) converting the product into a salt or in vivo hydrolysable ester thereof.

The compounds of formula (VIII) herein which are *inter alia* intermediates for the compounds of formula (I) as hereinbefore defined may be prepared by reacting a compound of formula (VI) with an N-acylating derivative of an acid of formula (XI):

$$R^1.\overset{*}{C}H.CO_2H \quad (\text{CH bearing } NHR^{11}) \qquad \text{(XI)}$$

wherein $R^{11}$ is an amino-protecting group and thereafter removing protecting group $R^{11}$.

Suitable amino-protecting groups $R^{11}$ are those

well-known in the art which may be removed under conventional conditions without disruption of the remainder of the molecule.

Examples of amino-protecting groups for $R^{11}$ include benzyl optionally substituted in the phenyl ring by one or two substituents selected from $C_{1-4}$ alkyl, $C_{1-4}$ alkoxy, trifluoromethyl, halogen or nitro; $C_{1-4}$ alkoxycarbonyl, for example tert-butoxycarbonyl; benzyloxycarbonyl optionally substituted as for benzyl above; allyloxycarbonyl; trityl or trichloroethoxycarbonyl.

Preferred examples of N-protecting groups within $R^{11}$ include those listed above which are removable under acid conditions optionally in the presence of a group IIb metal.

The intermediate compound of formula (VI) as hereinbefore defined may suitably be prepared by formylating a compound of formula (XII):

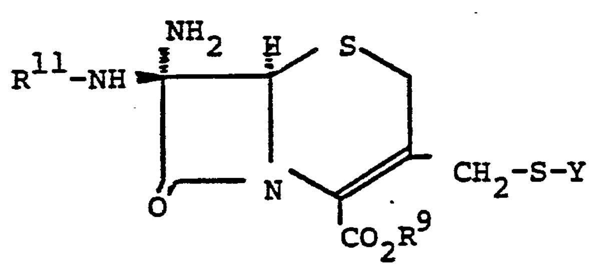

(XII)

wherein $R^9$, $R^{11}$ and Y are as hereinbefore defined and thereafter removing the protecting group $R^{11}$ and if

necessary, converting a group $R^9$ to a group $R^{10}$.

Suitable formylating agents and reaction conditions are as hereinbefore defined.

The compounds of the present invention may also suitably be prepared by reacting a compound of formula (XIII):

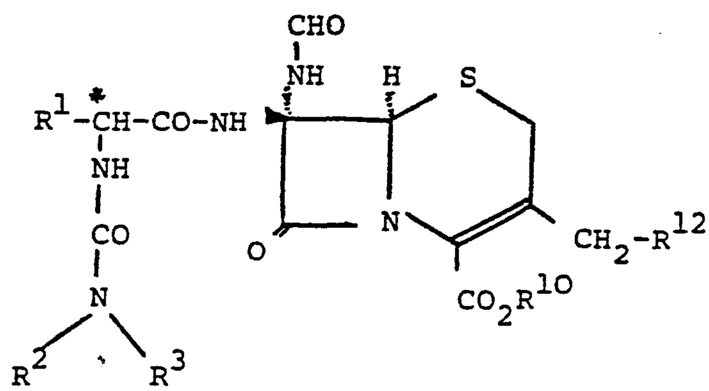

(XIII)

wherein $R^1$, $R^2$, $R^3$ and $R^{10}$ are as defined hereinbefore and wherein any reactive groups may be protected and $R^{12}$ is a leaving group; with a thiol of formula:

Y-SH

with the proviso that when $R^{12}$ is an acyloxy group $-CO_2R^{10}$ must be in the free acid form or a salt thereof.

Suitable leaving groups $R^{12}$ include halogen such as

iodide or bromide or an acyloxy groups such as, for example the acetyloxy group.

The thiol Y-SH may be reacted as the free compound or a salt with an alkali metal such as sodium or potassium. This reaction is desirably conducted in a solvent. For example, use can be made of water, or organic solvents inert to the starting compounds, such as dimethylformamide, dimethylacetamide, dioxane, acetone, alcohol, 1,2-dichloroethane, acetonitrile, dimethylsulfoxide or tetrahydrofuran, or mixtures thereof. The reaction temperature and time depend, among other factors, upon the starting compounds and solvent to be employed but generally the reaction is carried out at a selected temperature within the range of 0 to 100°C for a selected time of a few hours to several days. The reaction is desirably conducted between pH 3 and 7.

To prevent oxidation of the thio compounds it is advantageous to carry out the reaction in an inert gaseous atmosphere, eg nitrogen gas.

From the foregoing it will be appreciated that the compounds of (VI) and protected derivatives thereof are valuable intermediates and form another preferred aspect of the present invention.

Specific compounds within formula (VI) include the following or a salt thereof:

7β-Amino-3-[(1-carboxymethyl-1<u>H</u>-tetrazol-5-yl)

thiomethyl]-7α-formamidoceph-3-em-4-carboxylic acid bis-diphenylmethyl ester;

7β-Amino-3-[(1-carbamoylmethyl-1*H*-tetrazol-5-yl)thiomethyl]-7α-formamidoceph-3-em-4-carboxylic acid or the diphenylmethyl ester thereof;

7β-Amino-3-[(1-(2-dimethylaminoethyl)-1*H*-tetrazol-5-yl)thiomethyl]-7α-formamidoceph-3-em-4-carboxylic acid or the diphenylmethyl ester thereof;

and 7β-amino-3[(6-carbamoyl-2-methyl-5-oxo-4*H*-1,2,4-triazin-3-yl)thiomethyl]-7α-formamidoceph-3-em-4-carboxylate or the diphenylmethyl ester thereof.

The antibiotic compounds of the present invention are active against a wide range of gram negative and gram positive organisms including *E.coli* such as, for example ESS, JT4, JT425 and NCTC 10418; *Pseudomonas Spp.* such as *Ps.aeruginosa* for example 10662 and Dalgleish; *Serratia marcescens* US32; *Klebsiella aerogenes* A; *Enterobacter cloacae* N1; *P.mirabilis* such as, for example C977 and 889; *P.morganii*; *P.rettgeri*; *B.subtilis*; *Staph aureus* such as, for example Oxford and Russell; *N.catarrhalis* 1502; *Strep faecalis* I; *β-Haemolytic Strep* CN10. The MIC data included in the following examples is representative of the activity of the compounds of the present invention.

The following Examples illustrate the preparation and use of the compounds of the present invention.

Example 1

7β-Amino-3-[(1-carboxymethyl-1H-tetrazol-5-yl)thiomethyl]-7α-formamidoceph-3-em-4-carboxylic acid, disodium salt.

7β-Amino-7α-formamidocephalosporanic acid, trifluroacetic acid salt (600mg, 1.4mmol) in water (12.5ml) and acetone (5ml) was treated with saturated sodium hydrogen carbonate solution to pH 6.5. 5-mercapto-1*H*-tetrazol-1-acetic acid (278mg, 1.7mmol) was added and the pH adjusted to 3.5 with 5N hydrochloric acid. The solution was heated at 60°C under argon for 6h, cooled and the pH adjusted to 6.5. The solvents were evaporated and the residue chromatographed on Diaion HP20SS eluting with water. Lyophilization of the relevant fractions gave the title compound (385mg, 60%) $\nu_{max}$(KBr) 3300, 1760. 1600cm$^{-1}$.

Example 2

7β-[DL-2-(3,4-Diacetoxyphenyl)-2[4-ethyl-2,3-dioxopiperazin-1-yl)carbonylamino] acetamido]-7α-formamido-3-[(6-hydroxy-4-methyl-5-oxo-4H-1,2,4-triazin-3-yl)thiomethyl]ceph-3-em-4-carboxylic acid, disodium salt.

(a) 7β-Amino-3-[6-hydroxy-4-methyl-5-ox-4H-1,2,4-triazin-3-yl)thiomethyl]-7α-formamido ceph-3-em-4-carboxylic acid

7β-Amino-7α-formamidocephalosporanic acid trifluoro

acetic acid salt (0.836g, 1.953 mmol) was dissolved in water (32ml) and acetone (8ml) and 6-hydroxy-3-mercapto-4-methyl-5-oxo-4H-1,2-4-triazine (0.404g, 2.539mmol) added. The pH of the solution was adjusted to 3.5 and the solution heated at 60°C under argon for 3.25h and then allowed to stand at ambient temperature for 18 h. The pH was adjusted to 3.5, the reaction mixture concentrated to _ca_ 5 ml and chromatographed on Diaion HP20SS. Fractions containing the title compound were freeze-dried to afford a light yellow solid (0.680g), $\nu_{max}$(KBr) 3400, 300, 2890, 1755, 1700, 1670, and 1590 $cm^{-1}$, $\lambda_{max}$($H_2O$) 266nm (13,700), $\delta$[$(CD)_3SO$] 3.20-3.50br (4H, $NH_3$ and OH, exch.), 3.28 (3H,s,N,Me), 3.47 and 3.57 (together 2H, 2xABq, _J_16 and 14Hz respectively, 2-$CH_2$), 4.06 and 4.09 (together 2H, 2xABq, both _J_14Hz, $CH_2S$), 4.93 and 5.01 (together 1H, each s, 6-H), 8.04 and 8.29 (together 1H, each d, _J_ 1 and 11 Hz respectively, NHC_H_O, exch. to 2xs), 8.95 br and 9.11br [together 1H, d(_J_11Hz) and s respectively, N_H_.CHO,exch] and 12.45 br (1H,s, OH).

(b) Diphenylmethyl 7β-amino-7α-formamido-3-[(6-hydroxy-4-methyl-5-oxo-4H-1,2,4-triazin-3-yl)thiomethyl]ceph-3-em-4-carboxylate.

7β-Amino-3-[(6-hydroxy-4-methyl-5-oxo-4H-1,2,4-triazin-3-yl)thiomethyl]-7α-formamidoceph-3-em-4-carboxylic acid (828mg, 2mmol) in water (10ml) was cooled to 0-5°C and acidified to pH2 with 5M hydrochloric acid. The reaction mixture was concentrated to dryness _in vacuo_ and the residue redissolved in N,N-dimethylformamide (25ml) and the resulting mixture

treated with a large excess of diphenyldiazomethane in dichloromethane for 24 h. Glacial acetic acid (5 drops) was added and the mixture concentrated to low volume *in vacuo*. Ethyl acetate (50ml) and water (50ml) were added and the phases separated. The aqueous phase was further extracted with ethyl acetate (50ml), the extracts combined, washed with water (5x30ml), saturated brine (30ml), dried over anhydrous magnesium sulphate and evaporated to yield a dark red foam. Chromatography on silica gel 60 (230mesh ASTM), eluting with ethyl acetate, gave the title compound as a foam, which was solidified by trituration under ether to give a white solid (287mg, 25%; m.p. 135-140°C; $\nu_{max}(CHCl_3)$ 1780 and 1710cm$^{-1}$; $\delta(CDCl_3)$ 2.68 (2H, brs, $NH_2$) 3.33 (3H,s,$CH_3$), 3.35 and 3.53 (2H, ABq, *J*17.2Hz, 2-$H_2$), 3.88 and 4.30 (2H, ABq, *J*13.7Hz, 3-$CH_2$) 5.18 (1H, s, 6-H), 7.02 (1H, s, CH, $pH_2$), 7.20-7.61 (10H, m, aromatics) 8.18 (1H,s, CHO), 10.18-11.09 (1H, brs, OH).

(c) Diphenylmethyl 7β-[DL-2-(3,4-diacetoxyphenyl)-2-[(4-ethyl-2,3-dioxopiperazin-1-yl)carbonylamino]-7α-formamido-3-[(6-hydroxy-4-methyl-5-oxo-4H-1,2,4-triazin-3-yl)thiomethyl]ceph-3-em-4-carboxylate.

Diphenylmethyl 7β-amino-7α-formamido-3-[(6-hydroxy-4-methyl-5-oxo-4*H*-1,2,4-triazin-3-yl)thiomethyl]ceph-3-em-4-carboxylate (100mg;0.17mMole) in tetrahydrofuran (5ml) was stirred and treated with *N,N*-dicyclohexylcarbodiimide (39mg, 0.19mMole) at room temperature. To this mixture was added, dropwise, a solution of DL-2-(3,4-diacetoxyphenyl)-2-[(4-ethyl-2,3-dioxopiperazin-1-yl)carbonylamino]acetic acid (75mg, 0.17mMole) in

tetrahydrofuran (5ml). The mixture was stirred at room temperature for 24h and then the insolubles were removed by filtration, washed well with tetrahydrofuran and the filtrate evaporated to dryness *in vacuo* to yield a yellow foam. Chromatography on silica gel 60 (230mesh ASTM), eluting with 5% ethanol in ethyl acetate, gave the two, separated diastereoisomers as foams (total 124 mg, 70%):

Isomer 1: $\nu_{max}$ (KBr), 3260br, 1780, 1710, 1690sh, 1595, 1500, 1368, 1258 and 1180cm$^{-1}$; $\delta$[$(CD_3)_2CO$] 1.16 (3H, t, *J*7Hz, $NCH_2C\underline{H}_3$), 2.24 (6H, s, $2CH_3CO_2$'s). 2.84 and 3.08 (2H, ABq, *J* 8Hz, 2-$H_2$), 3.30 (3H, s, $NCH_3$), 3.48 (2H, q, *J*7Hz, $NC\underline{H}_2CH_3$), 3.55-3.82 and 3.86 - 4.14 (4H, 2m, $N(CH_2)N$), 3.85 and 4.58 (2H, ABq, *J*14Hz, 3-$CH_2S$), 5.28 (1H, s, 6-H), 5.78 (1H, d, *J* 8Hz, CH), 6.93 (1H, s, $C\underline{H}Ph_2$), 7.10 - 7.80 (13H, m, aromatics), 8.29 (1H, s, CHO), 8.48 (1H, brs, N$\underline{H}$CHO), 8.91 (1H, brs, CONH), 10.07 (1H, d, *J*8Hz, N$\underline{H}$CHCO), 11.34 (1H, brs, OH);

Isomer 2: $\nu_{max}$ ($CHCl_3$) 3240br, 1775, 1710, 1690sh, 1595, 1365, 1258, and 1180 cm$^{-1}$; $\delta$[$(CH_3)_2CO$] 1.13 (3H, t, *J*7Hz, $NCH_2C\underline{H}_3$), 2.25 (6H, s, $2CH_3CO_2$'s), 3.24-3.81 (9H, m, $C\underline{H}_2CH_3$, $NCH_2CH_2N$, $NCH_3$, and 2-$H_2$), 3.85-4.10 (2H, m, $NCH_2CH_2N$), 4.40 and 4.08 (2H, ABq, *J*14Hz, 3-$CH_2S$), 5.29 (1H, s,6-H), 5.84 (1H, d, *J*7Hz, CHCO), 6.94 (1H, s, e$\underline{m}$p$H_2$), 7.09-7.76 (13H, m, aromatics), 8.23 (1H, s, CHO), 8.60 (1H, brs, N$\underline{H}$CHO), 8.72 (1H, brs, CONH), 10.00 (1H, d, *J*7Hz, N$\underline{H}$CHCO), 11.36 (1H, brs, OH).

d) 7β-[DL-2-(3,4-diacetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid, disodium salt.

Diphenylmethyl 7β-[DL-2-(3,4-diacetoxyphenyl)-2-[(2,3-dioxo-4-ethylpiperazin-1-yl)carbonylamino]acetamido] -7α-formamido-3-[(6-hydroxy-4-methyl-5-oxo-4*H*-1.2.4-triazin-3-yl)thiomethyl]ceph-3-em-4-carboxylate (94mg, 0.1mMole) was stirred at room temperature in trifluoroacetic acid (5ml) until thin layer chromatography (silica gel; 5% ethanol in ethyl acetate) showed that no starting ester remained (*ca*. 0.5h), when the volatiles were removed *in vacuo*. The residue was azeotroped with chloroform (3x10ml), redissolved in tetrahydrofuran (5ml) and treated with 1.93M. sodium 2-ethylhexanoate in 4-methylpentan-2-one (0.2ml; 0.2mMole) followed by ether (50ml). The insoluble material (81mg, 99%) was collected by filtration, washed with ether and dried *in vacuo*; $\nu_{max}$(KBr) 3700-2500, 1775sh, 1765, 1703sh, 1680, 1560, 1400, 1370, 1204, 1185sh, and 1130cm$^{-1}$;$\delta D_2O$ 1.00-1.40 (3H, m, $NCH_2C\underline{H}_3$) 3.22 (6H, brs, $2CH_3CO_2$'s), .35 (3H, s, $NCH_3$), 3.35-4.10 (10H, m, $NCH_2CH_2NCH_2$,3-$CH_2S$ and 2-$H_2$), 5.22 (1H, s, 6-H), 5.45 (1H, s, CH), 7.20-7.55 (3H, m, aromatics), 8.09 (1H, brs, CHO).

Example 3

(i) 3-[(1-Carboxymethyl-1H-tetrazol-5-yl)thiomethyl]-7β-[2-[(4-ethyl-2,3-dioxopiperazin-1-yl)carbonylamino] -2-phenylacetamido]-7α-formamidoceph-3-em-4-carboxylic acid, disodium salt

7β-[2(4-Ethyl-2,3-dioxopiperazine-1-carbonylamino)-2-phenylacetamido]-7α-formamidocephalosporanic acid, sodium salt (0.088g, 0.131mmol) was taken up in water (6ml) and acetone (2ml) and 5-mercapto-1*H*-tetrazole-1-acetic acid (0.042 g, 0.252mmol) added. After adjusting the pH of the solution to 3.5, it was heated at 10°C under an argon atomosphere for 5h. The solution mixture was then concentrated *in vacuo* and taken up in a little saturated sodium hydrogen carbonate solution. The resulting solution was chromatographed on Diaion HP20SS and the fractions containing the desired product freeze-dried to afford the title compound as an amorphous pale-yellow solid (0.010 g) $\nu_{max}$(KBr) 3400, 3000, 1765, 1710, 1670 and 1620 $cm^{-1}$, $\delta(D_2O)$ *inter alia* 1.18 (t, *J* 7Hz, $N.CH_2.C\underline{H}_3$), 3.18 (ABq, *J* 17Hz, $2\text{-}C\underline{H}_2$), 3.49 (q, *J* 7Hz, $N.C\underline{H}_2.CH_3$), 3.6-3.8 (m, $N.CH_2.C\underline{H}_2.N$). 3.9-4.1 (m, $N.C\underline{H}_2.CH_2.N$ and $C.C\underline{H}H.S$), 4.33 (d, *J* 14Hz, $C.CH\underline{H}.S$), 5.21 (s, 6H), 5.47 (s, $N.CH_2.CO_2$), 5.48 (s, $Ph.C\underline{H}.NH$), 7.3-7.45 (m, aromatic H), and 8.10 and 8.41 (each s, $NH.C\underline{H}O$).

MIC (μg/ml) against *P. mirabilis* 889 is 0.25.

(ii) (a) Diphenylmethyl 3-[(1-diphenylmethoxycarbonylmethyl-1H-tetrazol-5-yl)thiomethyl]-7β-[D-2[(4-ethyl-2,3-dioxopiperazin-1-yl)carbonylamino]-2-phenylacetamido]-7α-formamidoceph-3-em-4-carboxylate.

Freshly prepared D-2-[(4-ethyl-2,3-dioxopiperazin-1-yl)carbonylamino]-2-phenylacetyl chloride, generated by treatment of the corresponding acid (0.092g, 0.289mmol) with oxalyl chloride (0.073g, 0.578mmol) in dry dichloromethane (3ml) containing *N,N*-dimethylformamide (1/10 drop) for 2 hour, was taken up in dichloromethane (3ml) and added to a stirred solution of diphenylmethyl 7β-amino-3[(1-diphenylmethoxycarbonylmethyl-1*H*-tetrazol-5-yl)thiomethyl]-7α-formamidoceph-3-em-4-carboxylate (0.108g, 0.145mmol), followed by pyridine (0.0114g, 0.145mmol). After 30 minutes the mixture was diluted with dichloromethane and washed successively with dilute aqueous sodium hydrogen carbonate, dilute hydrochloric acid, and saturated brine, dried ($MgSO_4$) and evaporated. The residue was chromatographed on silica gel to give the title compound (0.113g, 75%) as a gum, $\nu_{max}$($CH_2Cl_2$) 3270, 3170, 3050, 1785, 1745, 1715 and 1690cm$^{-1}$, δ[$(CD_3)_2CO/D_2O$] *inter alia* 1.18(3H,t,*J* 7.5Hz, N.$CH_2$.$\underline{CH_3}$), 3.13 and 3.27 (2H,ABq, *J*16Hz, 2-$CH_2$), 3.53(2H, q, *J* 7.5Hz, N.$\underline{CH_2}$.$CH_3$), 3.6-3.8 and 4.0-4.15(each 2H,m,$NCH_2CH_2N$), 4.27 and 4.35 [together 1H, high field half of 3-$CH_2$S ABq (*J*13.5Hz), minor and major rotamer respectively],4.48 and 4.52 [together 1H, low field half of 3-$CH_2$S ABq (*J*13.5Hz), major and minor rotamer respectively], 5.28 and 5.35 (together 1H, each s, 6-$\underline{H}$), 5.54(2H,s,$NCH_2CO_2$), 5.68(1H,s,NHC$\underline{H}$CO), 6.90, 6.92, 6.94 and 7.00 (together 2H, each s, 2xC$\underline{H}$$Ph_2$), 7.2-7.7(15 H, m, aromatics), and 8.19, 8.21, 8.27 and 8.53 (together 1H, each s, NHC$\underline{H}$O).

(b) <u>3-[(1-Carboxymethyl-1H-tetrazol-5-yl)thiomethyl]-7β-[D-2-[(4-ethyl-2,3-dioxopiperazin-1-</u>

yl)carbonylamino]-2-phenylacetamido]-7α-formamidoceph-3-em-4-carboxylic acid, disodium salt.

Diphenylmethyl 3[(1-diphenylmethoxycarbonylmethyl-1H-tetrazol-5-yl)thiomethyl]-7β-[D-2[(4-ethyl-2,3-dioxopiperazin-1-yl)carbonylamino]-2-phenylacetamido]-7α-formamidoceph-3-em-4-carboxylate (0.105g) was taken up in trifluoroacetic acid (5ml) and the solution allowed to stand at room temperature. After 15 minutes the solvent was evaporated *in vacuo* and the residue taken up in saturated aqueous sodium hydrogen carbonate. This solution was chromatographed on Diaion HP20SS to give the title compound (0.053g) as a white solid, $\nu_{max}$ (KBr) 3400, 2980, 1770, 1720, 1710, 1680, 1620 and 1490cm$^{-1}$, δ($D_2O$) 1.18(3H,t,*J*7Hz, N.$CH_2\underline{CH_3}$), 3.01 and 3.03 [together 1H, high field half of 2-$CH_2$ ABq, (*J*16.5Hz) major and minor rotamer respectively], 3.40 and 3.43 [together 1H, low field half of 2-$CH_2$ ABq (*J*16.5Hz), major and minor rotamer respectively], 3.50 (2H, q, *J*7Hz, $NC\underline{H}_2.CH_3$), 3.6-3.8 (2H,m,$NCH_2C\underline{H}_2N$), 3.9-4.1 (3H, m, $NC\underline{H}_2$ $CH_2N$ and high field half of 3-$CH_2S$), 4.33 and 4.39 [together 1H, low field half of 3-$CH_2S$ ABq (*J*13Hz), major and minor rotamer respectively], 4.98(2H, s, $NCH_2CO_2$), 5.23(1H,s,6-H), 5.50 (1H,s,NHC$\underline{H}$CO), 7.3-7.6(5H,m,Ph), 8.11 and 8.43 (together 1H, each s, NHC$\underline{H}$O, major and minor rotamer respectively).

MIC (µg/ml) against *P.Mirabilis* 889 is 0.06.

Example 4

Diphenylmethyl 7β-amino-3[(1-diphenylmethoxycarbonylmethyl-1-H-tetrazol 5-yl)thiomethyl]-7α-formamidoceph-3-em-4-carboxylate.

7α-Amino-3-[(1-carboxymethyl-1H-tetrazol-5-yl) thiomethyl]-7α-formamidoceph-3-em-4-carboxylic acid, di-sodium salt (120mg) was dissolved in water (2ml) and the pH adjusted to 2 by the addition of 5N hydrochloric acid. The solvent was evaporated and the residue dried in vacuo. Acetonitrile (10ml) was added, followed by excess diphenyldiazomethane. After 12 days, the mixture was treated with acetic acid (5 drops) for 1h. The mixture was poured into ethyl acetate-water and the organic layer separated, washed with saturated aqueous sodium hydrogen carbonate solution, brine, dried and evaporated. The residue was chromatographed on silica gel 60 (<230 mesh ASTM) to give the title compound (59mg) (31%); $\nu_{max}$. 3400br, 1775, 1755sh, 1710, 1690 $cm^{-1}$; δ($CDCl_3$) 2.28 and 2.4 (together 2H, broad singlets, minor and major rotamers respectively, $NH_2$, exch. $D_2O$), 3.54 (2H, s, ring S-$CH_2$), 4.22 and 4.36 (2H, ABq, *J* 13.3Hz, $CH_2$-S-), 5.02 and 5.11 (2H, ABq, *J* 17.6Hz, N-$CH_2$), 5.13 (1H, s, 6H), 6.3 and 6.42 (together 1H, d, *J* 12Hz, and s, minor and major rotamers respectively, NH, exch. $D_2O$), 6.92 (1H, s, $CHPh_2$), 6.95 (1H, s, $CHPh_2$), 7.21-7.58 (20H, m), 8.23 and 8.4 (together 1H, s and d J = 12 Hz, major and minor rotamer respectively, latter becomes s on $D_2O$ exch).

Example 5

7β[D-2-[(4-Ethyl-2,3-dioxopiperazin-1-yl)carbonylamino]-2-phenylacetamido]-7α-formamido-3-[(6-hydroxy-4-methyl-5-oxo-4H-1,2,4-triazin-3-yl)thiomethyl]ceph-3-em-4-carboxylic acid, disodium salt.

(a) Diphenylmethyl 7β-[D-2-[(4-ethyl-2,3-dioxopiperazin-1-yl) carbonylamino]-2-phenylacetamido]-7α-formamido-3-[(6-hydroxy-4-methyl-5-oxo-4H-1,2,4-triazin-3-yl)thiomethyl]ceph-3-em-4-carboxylate.

Freshly prepared D-2-[(4-ethyl-2,3,dioxopiperazin-1-yl) carbonylamino]-2-phenylacetylchloride, generated by the treatment of the corresponding acid (0.066g, 0.207mmol) with oxalyl chloride (0.053g, 0.414mmol) and *N*,*N*-dimethylformamide (1 drop) in dry dichloromethane (4ml) for 1 hour, was taken up in dichloromethane (1ml) and added to a stirred solution of diphenylmethyl 7β-amino-7α-formamido-3[(6-hydroxy-4-methyl-5-oxo-4H-1,2,4-triazin-3-yl)thiomethyl]ceph-3-em-4-carboxylate (0.080g, 0.138mmol) in dichloromethane (3ml) containing pyridine (0.011g, 0.138mmol). After 90 minutes, the mixture was washed successively with dilute aqueous sodium hydrogen carbonate, dilute hydrochloric acid, and saturated brine, dried ($MgSO_4$) and evaporated. The residue was chromatographed on silica gel to give the title compound (0.071g, 58%) as a cream coloured solid, $\nu_{max}$ (Nujol) 3250, 1775, 1715, 1705, and 1680cm$^{-1}$, δ[$(CD_3)_2CO$] 1.19(3H,t,*J*7Hz,$NCH_2C\underline{H}_3$), 3.33(3H,s,$NCH_3$), 3.25 - 3.60 (together 4H, m, 2-$CH_2$ and N.$C\underline{H}_2$.$CH_3$), 3.60-3.85(2H,m, N.$CH_2$.$C\underline{H}_2$.N), 3.95-4.15(2H,m,$NC\underline{H}_2CH_2N$), 4.16 and 4.31 (2H, ABq, *J*14Hz, 3-$CH_2S$), 5.27(1H,s,6-H), 5.77 (1H,d,*J* 7Hz,Ph.C$\underline{H}$.NH), 6.94(1H,s,C$\underline{H}$.$Ph_2$),

7.2-7.7(15H,m,aromatics), 8.29 and 8.56 (together 1H, each s, NH.CHO, major and minor rotamer respectively), 8.66 and 8.71 (each 1H, br, CONHC and NH.CHO), 10.04 (1H,d,J 7Hz, CH.NH.CO) and 11.52(1H,br,OH), (addition of $D_2O$ caused the signals at 8.66, 8.71, 10.04 and 11.52 to disappear whilst that at 5.77 collapsed to a s).

(b) 7β-[D-2-[(4-Ethyl-2,3-dioxopiperazin-1-yl)carbonylamino]-2-phenylacetamido]-7α-formamido-3-[(6-hydroxy-4-methyl-5-oxo-4H-1,2,4-triazin-3-yl)thiomethyl]ceph-3-em-4-carboxylic acid, disodium salt.

Diphenylmethyl 7β-[D-2-[(4-ethyl-2,3-dioxopiperazin-1-yl)carbonylamino]-2-phenylacetamido]-7α-formamido-3-[(6-hydroxy-4-methyl-5-oxo-4H-1,2,4-triazin 3-yl)thiomethyl]ceph-3-em-4-carboxylate (0.063g, 0.072mmol) was taken up in trifluoroacetic acid (5ml) and allowed to stand at room temperature. After 45 minutes the solvent was evaporated and residue taken up in saturated aqueous sodium hydrogen carbonate. The resulting cloudy solution was chromotographed on Diaion HP2OSS to afford the title compound (0.007g) as a white solid, $\nu_{max}$ (KBr) 3400, 3300, 3000, 1770, 1710, 1680 and 1590cm$^{-1}$, δ($D_2O$) 1.18 (3H,t,J7Hz, $N.CH_2.CH_3$), 2.97 and 3.01 (together 1H, each d, J16.5Hz, 2-CHH, major and minor rotamer respectively), 3.33-3.45(together 4H,m,$NCH_3$ and 2CHH, sharp s at 3.39 discernable), 3.48(2H,q,J7Hz, $N.CH_2CH_3$) 3.53-3.73 and 3.85-4.10 (each 2H,m,$NCH_2CH_2N$), 3.76 and 4.39 (2H, ABq, J14.1Hz, $3-CH_2S$), 5.17 and 5.22(together 1H, each s, 6-H, minor

and major rotamer respectively), 5.45 and 5.50 (together 1H, each s, Ph.C<u>H</u>.NH, minor and major rotamer respectively), 7.30-7.60 (5H,m,aromatics), and 8.11 and 8.44 (together 1H, each s, major and minor rotamer respectively).

MIC (μg/ml) against <u>P.mirabilis</u> 889 is <0.06.

<u>Example 6</u>

<u>7β-[D-2(4-Acetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid, disodium salt.</u>

<u>(a)</u> <u>Diphenylmethyl 7β-[D-2(4-acetoxyphenyl)-2[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylate.</u>

Freshly prepared [D-2-(4-acetoxyphenyl)-2-[(4-ethyl-2,3-dioxopiperazin-1-yl)carbonylamino]]acetyl-chloride, generated from the corresponding acid (0.165g, 0.438mmol) as described in Example 5(a) was added to diphenylmethyl 7β-amino-7α-formamido-3[(6-hydroxy- 4-methyl-5-oxo-4H-1,2,4-triazin-3-yl)thiomethyl]ceph-3-em-4-carboxylate (0.060g, 0.103mmol) in dichloromethane (2ml) containing pyridine (0.008g, 0.103mmol). After 2 hour, the mixture was worked up as in Example 5 to give the title compound (0.071g, 73%) as a white solid, $\nu_{max}$ (Nujol) 3500, 3270, 1785, 1760,

1720,1710,1685 and 1585cm$^{-1}$, δ[$(CD_3)_2$] inter alia 1.18 (t, J6.5Hz,$N.CH_2CH_3$), 2.25 (s, $OCOCH_3$), 3.24 and 3.36 (ABq, J18Hz, S-$CH_2$), 3.34 (s,$NCH_3$), 3.45-3.60 (m,N.$CH_2$.$CH_3$), 3.65-3.75 and 4.0-4.14 (each m, $NCH_2CH2N$), 4.16 and 4.35 (ABq, J13Hz, 3-$CH_2S$), 5.29 (s, 6-H), 5.76 (d,J10Hz, PhCHNH), 6.95 (s,CH.$Ph_2$), 7.05-7.90 (m, aromatics, NH.CHO, CONHC, and OH), 8.30 and 8.56 (each s, NH.CHO, major and minor rotamer respectively), and 10.07 (d,J10Hz,CH.NH.CO).

b) 7β-[D-2(4-Acetoxyphenyl)-2-[(4-ethyl-2,3-dioxo-piperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid, disodium salt

Diphenylmethyl 7β-[2-(4-acetoxyphenyl)-2[(4-ethyl-2,3-dioxopioerazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylate (0.063 g) was treated with trifluroroacetic acid (2 ml) and worked up as described in Example 5b to give the title compound (20 mg) as a white solid, $\nu_{max}$ (KBr) 3380, 3000, 1760, 1710, 1670 and 1590 cm$^{-1}$, δ($D_2O$) 1.18 (3H, t, J 7.5Hz, N.$CH_2CH_3$), 2.33 (3H, s, $OCOCH_3$), 2.97 and 2.99 (together 1H, each d, J 17Hz, higher field half of ABq for 2-$CH_2$, major and minor rotamer respectively), 3.41 (1H, d, J 17Hz, lower field half of ABq for 2-$CH_2$), 3.42 (3H, s, N.$CH_3$), 3.51 (2H, q, J 7.5Hz, N.$CH_2CH_3$), 3.60-3.80 and 3.90-4.10 (each 2H, m, N.$CH_2$.$CH_2$.N), 3.77 and 4.40 (2H, ABq, J 14Hz, 3-$CH_2S$), 5.18 and 5.22 (together 1H, each s, 6-H, minor and

major rotamer respectively), 5.49 and 5.53 (together 1H, each s, NH.C<u>H</u>.CO, minor and major rotamer respectively), 7.17 and 7.59 (4H, ABq, *J* 8.5Hz, aromatics), and 8.12 and 8.45 (together 1H, each s, NHC<u>H</u>O, major and minor rotamer respectively).

MIC (µg/ml) against *P. mirabilis* 889 is 0.5.

## Example 7

**7β-[DL-2-[(4-Ethyl-2,3-dioxopiperazin-1-yl)carbonylamino]-2-(thien-2-yl)acetamido]-7α-formamido-3-[(6-hydroxy-4-methyl-5-oxo-4H-1,2,4-triazin-3-yl)thiomethyl]ceph-3-em-4-carboxylic acid, disodium salt**

**(a) Diphenylmethyl 7β-[DL-2-[(4-ethyl-2,3-dioxopiperazin-1-yl)carbonylamino]-2-(thien-2-yl)acetamido]-7α-formamido-3-[(6-hydroxy-4-methyl-5-oxo-4H-1,2,4-triazin-3-yl)thiomethyl]ceph-3-em-4-carboxylate**

Freshly prepared [DL-2(4-ethyl-2,3-dioxopiperazin-1-yl)carbonylamino]-2-(thien-2-yl)acetylchloride, generated from the corresponding acid (0.131 g, 0.403 mmol) as described in Example 5(a) was added to diphenylmethyl 7β-amino-7α-formamido-3[(6-hydroxy-4-methyl-5-oxo-4H-1,2,4-triazin-3-yl)thiomethyl]ceph-3-em-4-carboxylate (0.116 g, 0.2 mmol) in dichloromethane (2 ml) containing pyridine (0.016 g, 0.2 mmol). After 30 min., work up as in Example 5(a) gave the title compound (0.101 g, 57%) as a pale gum,

δ[$(CD_3)_2CO$] 1.17 and 1.18 (together 3H, each t, J 7Hz, $N.CH_2C\underline{H}_3$), 2.85-3.05 (1H, 2-CH<u>H</u>, obscured by HOD in solvent), 3.25-3.40 (1H, 2-CH<u>H</u>, partially obscured by $N-CH_3$), 3.33 and 3.35 (together 3H, each s, $N.CH_3$), 3.4-3.6 (2H, m, $N.C\underline{H}_2.CH_3$), 3.6-3.8 (2H, m, $N.CH_2.C\underline{H}_2.N$), 3.95-4.20 (3H, m, $N.C\underline{H}_2.CH_2.N$ and upfield half of $3-CH_2$ ABq), 4.32 and 4.41 [(together 1H, lower field half of $3-CH_2S$ ABq (J 14Hz)], 5.30 and 5.32 (together 1H, each s, 6-H), 6.06 and 6.08 (together 1H, each d, J 9Hz, NH.C<u>H</u>.CO), 6.9-7.7 (16H, m, aromatics, C<u>H</u>.$Ph_2$, N<u>H</u>CHO and CO.NH.C), 8.27, 8.29, 8.47 and 8.57 (together 1H, each s, NH.C<u>H</u>O), and 9.96 (1H, br, d, J 9Hz, CH.N<u>H</u>.CO).

(b) <u>7β-[DL-2[(4-Ethyl-2,3-dioxopiperazin-1-yl)carbonyl amino]-2-(thien-2-yl)acetamido]-7α-formamido-3-[(6-hydroxy-4-methyl-5-oxo-4H-1,2,4-triazin-3-yl) thiomethyl]ceph-3-em-4-carboxylic acid, disodium salt.</u>

Diphenylmethyl 7-β[DL-2[(4-ethyl-2,3-dioxopiperazin-1-yl)carbonylamino]-2-(thien-2-yl)acetamido]-7α-formamido-3[(6-hydroxy-4-methyl-5-oxo-4H-1,2,4-triazin-3-yl)thiomethyl]ceph-3-em-4-carboxylate (0.092g) was treated with trifluoroacetic acid (2ml) for 10 minutes and the solution worked up as described in Example 5(b) to give the title compound (0.040g) as a white solid, $\nu_{max}$ (Nujol) 3500, 3260, 1775, 1710, 1680, and 1590$cm^{-1}$, δ($D_2O$) 1.19 (3H,t,J 7.5Hz, $N.CH_2.C\underline{H}_3$), 3.15 and 3.20 (together 1H, high-field half of $2-CH_2$ ABq, J18Hz), 3.44 and 3.46 (together 3H, each s, $NCH_3$),

3.40-3.55(1H,low-field half of 2-$CH_2$ ABq, obscured by N.$CH_3$ and N.C<u>H</u>$_2$.$CH_3$), 3.51(2H,q,<u>J</u>7.5Hz, N.C<u>H</u>$_2$.$CH_3$), 3.6-3.9 (3H,m,N.$CH_2$.C<u>H</u>$_2$,N and high field half of 3-$CH_2$S ABq), 3.95-4.15(2H,m,N.C<u>H</u>$_2$$CH_2$.N), 4.43 and 4.48 (together 1H, lower-field half of 3-$CH_2$S ABq, <u>J</u>14Hz), 5.19, 5.21, 5.24 and 5.63 (together 1H, each s, 6-H), 5.78, 5.82, 5.86 and 6.14 (together 1H, each s, NH.C<u>H</u>.CO), 7.0-7.1, 7.15-7.30 and 7.4-7.55 (together 3H, each m, aromatics), and 8.11, 8.13, 8.22 and 8.46 (together 1H, each s, NHC<u>H</u>O).

MIC (µg/ml) against <u>P.mirabilis</u> 889 is 0.06

<u>Example 8</u>

<u>7β-[DL-2[(4-Ethyl-2,3-dioxopiperazin-1-yl)carbonylamino]-2-(2-fluorophenyl)acetamido]-7α-formamido-3-[(6-hydroxy-4-methyl-5-oxo-4H-1,2,4-triazin-3-yl)-thiomethyl]ceph-3-em-4-carboxylic acid, disodium salt.</u>

<u>(a) Diphenylmethyl 7β-[DL-2-[(4-ethyl-2,3-dioxopiperazin-1-yl)carbonylamino]-2-(2-fluorophenyl)acetamido]-7α-formamido-3-[(6-hydroxy-4-methyl-5-oxo-4H-1,2,4-triazin-3-yl)-thiomethyl]ceph-3-em-4-carboxylate.</u>

Freshly prepared [DL-2(4-ethyl-2,3-dioxopiperazin-1-yl)carbonylamino]-2-(2-fluorophenyl)acetyl chloride, generated from the corresponding acid (0.138g, 0.409mmol) as described in Example 5(a) was added to diphenylmethyl

7β-amino-7α-formamido-3[(6-hydroxy-4-methyl-5-oxo-4H-1,2,4-triazin-3-yl)thiomethyl]ceph-3-em-4-carboxylate (0.084g, 0.145mmol) in dichloromethane (3ml) followed by pyridine (0.0115g, 0.145mmol). After 15 minutes the mixture was worked up as described in Example 5(a) to provide the title compound (0.113g, 87%) as a white solid, $\nu_{max}$ ($CH_2Cl_2$) 3250, 3030, 1785, 1720, 1710, and 1690cm$^{-1}$, δ[$(CD_3)_2CO$] *inter alia* 1.18 and 1.19 (t, *J*7H*z*, $NCH_2CH_3$), 3.34 and 3.36(each s, $NCH_3$), 3.4-3.6 (m, $NCH_2CH_3$ and 2-$CH_2$), 3.6-3.8 (m, $NCH_2CH_2N$), 3.9-4.2(m, $NCH_2CH_2N$), 4.07, 4.18, 4.33 and 4.44 (2xABq, *J*14Hz, 3-$CH_2S$), 5.30, 5.31 and 5.33 (each s, 6-H), 5.98 (d,*J*8Hz, NHCHCO), 6.94 and 6.96 (each s, $CHPh_2$), 7.1-7.7 (m, aromatics), 8.17, 8.27, 8.29 and 8.57(each s, NHCHO), 10.0 br (CONHCH).

(b) 7β-[DL-2[(4-Ethyl-2,3-dioxopiperazin-1-yl)carbonylamino]-2-(2-fluorophenyl)acetamido]-7α-formamido-3-[(6-hydroxy-4-methyl-5-oxo-4H-1,2,4-triazin-3-yl)thiomethyl]ceph-3-em-4-carboxylic acid, disodium salt.

Diphenylmethyl 7β-[DL-2[(4-ethyl-2,3-dioxopiperazin-1-yl)carbonylamino]-2-(2-fluorophenyl)acetamido]-7α-formamido-3-[(6-hydroxy-5-oxo-4H-1,2,4-triazin-3-yl]thiomethyl]ceph-3-em-4-carboxylate (0.103g) was treated with trifluoroacetic acid. After 15 minutes the solution was worked up as described in Example 5(b) to give the title compound (0.044g) as a white powder, $\nu_{max}$ (KBr) 3420, 3000, 1770, 1720, 1700, 1680, 1610 and 1590cm$^{-1}$, δ($D_2O$) 1.19(3H,t,*J*8Hz, $NCH_2CH_3$), 3.09 and 3.16

(together 1H, high field half of 2-$CH_2$ABq, *J*18Hz), 3.42 and 3.44 (together 3H, each s, N-$CH_3$), 3.51(2H, q, *J*8Hz, NC*H*$_2$CH$_3$), 3.4-3.5 (1H,low field half of 2-$CH_2$ ABq, obscured by $NCH_3$ and NC*H*$_2$CH$_3$), 3.60-3.85(3H,m,NCH$_2$C*H*$_2$N and high field half of 3-$CH_2$S ABq), 3.9-4.1(2H,m,NC*H*$_2$CH$_2$N), 4.43 and 4.46 (together 1H, low field half of 3-$CH_2$S ABq, *J*15Hz), 5.17, 5.20, 5.23 and 5.43(together 1H, each s, 6-H), 5.77, 5.81, 5.84 and 5.93 (together 1H, each s, NHC*H*CO), 7.1-7.6(4H,m,aromatics), 8.08, 8.12, 8.24 and 8.45 (together 1H, each s, NHC*H*O).

MIC (μg/ml) against *P.mirabilis* 889 is 0.5.

## Example 9

**7β-[D-2(3,4-Diacetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid, disodium salt.**

**(a) Diphenylmethyl 7β-[D-2-(3,4-diacetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylate.**

Freshly prepared D-2(3,4-diacetoxyphenyl)-2-[(4-ethyl-2,3-dioxopiperazin-1-yl)carbonylamino]acetyl chloride, generated from the corresponding acid (0.107g, 0.246mmol) as described in Example 5(a) was added to diphenylmethyl

7β-amino-7α-formamido-3-[(6-hydroxy-4-methyl-5-oxo-4H-1,2,4-triazin-3-yl)thiomethyl]ceph-3-em-4-carboxylate (0.095g, 0.164mmol) in dichloromethane (4ml), followed by pyridine (0.013g, 0.164mmol). After 90 minutes the mixture was worked up as described in Example 5(a) to afford the title compound (0.094g, 58%) as a white solid, $\nu_{max}$ ($CHCl_3$) 3280, 3000, 1770, 1720, 1710 and 1685$cm^{-1}$, δ[$(CD_3)_2CO$] 1.17 (3H,t,J7Hz,$NCH_2CH_3$), 2.24 (6H,s,$2xOCOCH_3$), 2.8-3.1(1H, high field half of 2-$CH_2$ ABq, obscured by HOD in solvent), 3.1-3.3 (1H,m,low-field half of 2-$CH_2$), 3.33(3H,s,NMe), 3.4-3.6(2H,m,$NCH_2CH_3$), 3.6-3.8 and 3.9-4.1(each 2H,m, $NCH_2CH_2N$), 4.13 and 4.37(2H, ABq, J14Hz, 3-$CH_2S$), 5.28(1H,s,6-H), 5.72(1H,s,NHCHCO), 6.90 and 6.92 (together 1H, each s, $CHPh_2$), 7.1-7.7(11H,m,aromatics and NHCHO), 8.02(1H,s,CONHC), 8.29 and 8.45 (together 1H, each s, NHCHO, major and minor rotamer respectively) (addition of $D_2O$ caused the 2-$CH_2$'s to simplify to 2xABq (J16Hz)].

(b) 7β-[D-2(3,4-Diacetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid, disodium salt

Diphenylmethyl 7β-[D-2(3,4-diacetoxyphenyl)-2-[(4-ethyl-2,3-dioxo piperazin-1-yl)carbonylamino]acetamido]-7α-formamido--3-](6-hydroxy-4-methyl-5-oxo-4H-1,2,4-triazin-3-yl 3-yl)thiomethyl]ceph-3-em-4-carboxylate (0.088g) was treated with trifluoroacetic acid (2ml). After 10 minutes the solution was worked up as in Example 5(b)

to give the title compound (0.016g) as a white solid, $\nu_{max}$ (KBr) 3420, 2980, 1760, 1720, 1710, 1670 and 1590cm$^{-1}$, $\delta$($D_2O$) 1.18(3H,t,J7Hz, $NCH_2C\underline{H}_3$), 2.30 and 2.31 (each 3H,s, 2x$OCOCH_3$), 2.89 and 2.93 (together 1H, each d, J16.5Hz, high field half of 2-$CH_2$), 3.25-3.40(1H,low field half of 2-$CH_2$, obscured by NMe), 3.41(3H,s,NMe), 3.50(2H,q,J7Hz, $NC\underline{H}_2CH_3$), 3.55-3.80 (2H,m,$NCH_2C\underline{H}_2N$), 3.80 and 4.39(2H,ABq, J14Hz, 3-$CH_2S$), 5.22 and 5.24 (together 1H, each s, 6-H), 5.50 and 5.52(together 1H, each s, NHC$\underline{H}$CO), 7.2-7.6 (3H,m, aromatics) and 8.12 and 8.45 (togethr 1H, each s, NHC$\underline{H}$O, major and minor rotamer respectively).

MIC (µg/ml) against P. mirabilis 889 is 0.06

Example 10

3-[(1-Carboxymethyl-1H-tetrazol-5-yl)thiomethyl]-7β-[D-2(3,4-diacetoxyphenyl)-2-[(4-ethyl-2,3-dioxopiperazin-1-yl)carbonylamino]acetamido]-7α-formamidoceph-3-em-4-carboxylic acid, disodium salt.

(a) Diphenylmethyl 3-[(1-diphenylmethoxycarbonylmethyl-1H-tetrazol-5-yl)thiomethyl]-7β [D-2(3,4-diacetoxyphenyl)-2-[(4-ethyl-2,3-dioxopiperazin-1-yl)carbonylamino]acetamido-7α-formamidoceph-3-em-4-carboxylate.

Freshly prepared D-2(3,4-diacetoxyphenyl)-2-[(4-ethyl-2,3-dioxopiperazin-1-yl)carbonylamino]acetyl chloride, generated by the treatment of the corresponding acid (0.047g, 0.108mmol)

with oxalyl chloride (0.027g, 0.216mmol) in dry dichloromethane (5ml) containing *N*,*N*-dimethylformamide (1/10 drop) for 90 minutes, was taken up in dichloromethane (2ml) and added to a stirred solution of diphenylmethyl 7β-amino-3[(1-diphenylmethoxycarbonylmethyl-1H-tetrazol-5-yl)thiomethyl]-7α-formamidoceph-3-em-4-carboxylate (0.054g, 0.072mmol) followed by pyridine (0.006g, 0.072mmol). After 90 minutes the mixture was diluted with dichloromethane and washed successively with dilute aqueous sodium hydrogen carbonate, dilute hydrochloric acid and saturated brine, dried ($MgSO_4$) and evaporated. The residue was chromatographed on silica gel to give the title compound (0.046g, 55%) as a yellow foam, $\nu_{max}$ ($CHCl_3$) 3290,3000, 1770, 1745, 1715 and 1690$cm^{-1}$, δ[$(CD_3)_2CO$] 1.15(3H,t,*J* 7.5Hz, $NCH_2C\underline{H}_3$), 2.21 and 2.22 (each 3H,2s,2 x $OCOCH_3$), 2.91 and 3.20 (2H,ABq, *J*17Hz, 2-$CH_2$), 3.49(2H,2xq, *J* 7.5Hz, $NC\underline{H}_2CH_3$), 3.6 - 3.8 and 3.95-4.10(each 2H,m,N.$CH_2$.$CH_2$N), 4.13 and 4.65(2H,ABq,*J*13Hz,3-$CH_2$S), 5.22(1H,s,6-H), 5.46 and 5.55(2H,ABq,*J*17.5 Hz, $NCH_2CO$), 5.73(1H,d,*J*7Hz, NHC<u>H</u>CO), 6.86, 6.89 and 6.93 (together 2H,each s, 2xC<u>H</u>$Ph_2$), 7.15-7.70(24H,m,aromatics and N<u>H</u>.CHO), 8.02 (1H,s,CONHC) 8.27 and 8.52 (together 1H,each s, NHC<u>H</u>O, major and minor rotamer respectively), and 10.08 (1H,d,*J*7Hz,CH.N<u>H</u>.CO), (addition of $D_2O$ caused the signal 10.08 to disappear whilst the signal centered at and 5.73 collapsed to s).

<u>(b) 3-[1-Carboxymethyl-1H-tetrazol-5-yl)thiomethyl-7β-[D-2(3,4-diacetoxyphenyl)-2-[(4-ethyl-2,3-dioxopiperazin-1-yl)carbonylamino]acetamido]-7α-formamidoceph-3-em -4-carboxylic acid, disodium salt</u>

Diphenylmethyl 3-[(1-diphenylmethoxycarbonylmethyl-1H-tetrazol-5-yl)thiomethyl]-7β-[D-2(3,4-diacetoxyphenyl)-2[(4-ethyl-2,3-dioxopiperazin-1-yl)carbonylamino acetamido]-7α-formamidoceph-3-em-4-carboxylate (0.040g) was taken up in trifluoroacetic acid (2ml) and the resulting solution allowed to stand at room temperature. After 10 minutes the solvent was removed *in vacuo* and the residue taken up in saturated aqueous sodium hydrogen carbonate. This solution was chromatographed on Diaion HP20SS to give the title compound (0.015g) as a white solid, $\nu_{max}$ (Nujol) 3300, 1775, 1765, 1720, 1710, 1690, 1675 and 1625cm$^{-1}$, $\delta(D_2O)$ 1.18(3H,t,*J*7.5Hz, $NCH_2CH_3$),2.34(6H,s,2x$OCOCH_3$), 2.91 and 2.95 [togethr 1H, high field half of 2-$CH_2$ ABq(*J*17.5Hz), major and minor rotamer respectively], 3.34 and 3.37 [together 1H, low field half of 2-$CH_2$ ABq (*J*17.5Hz), major and minor rotamer respectively], 3.51 (2H,q,*J*7.5Hz,$NCH_2CH_3$), 3.6-3.75(2H,m,$NCH_2CH_2N$), 3.9-4.1 (3H,m,$NCH_2CH_2N$ and high field half of 3-$CH_2S$ ABq), 4.37 and 4.43[together 1H, low field half of 3-$CH_2S$ ABq, (*J*14Hz), major and minor rotamer respectively],5.00 (2H,ABq, $NCH_2CO_2$), 5.23(1H,s,6-H), 5.54(1H,s, NHCHCO), 7.3-7.6(3H,m,aromatics), and 8.09, 8.12 and 8.45(1H, each s, NHCHO).

MIC (μg/ml) against *P.mirabilis* 889 is 0.06

Example 11

(a) 7β-Amino-3-[(1-carbamoylmethyl-1H-tetrazol-5-yl) thiomethyl]-7α-formamidoceph-3-em-4-carboxylic acid, sodium salt.

7β-Amino-7α-formamidocephalosporanic acid, trifluoroaetic acid salt (516mg, 1.2mmol) in water (18ml) and acetone (6ml) was treated with saturated sodium hydrogen carbonate solution to pH 6.5. 5-mercapto-1*H*-tetrazole-1-acetamide (384mg; 2.4mmol) was added and the pH adjusted to 3.5. The solution was heated at 60°C under argon for 6h, cooled and the pH adjusted to 6.5. The solvents were evaporated and the residue chromatographed on Diaion HP20SS eluting with water. Lyophilization of the relevant fractions gave the title compound (338mg, 65%); $\delta(D_2O)$ 3.35 and 3.69 (2H, ABq, *J*17Hz, 2-$H_2$), 4.02 and 4.28 (2H, ABq, *J*13Hz, $CH_2S$), 5.12 (1H, s, C-6), 5.28 (2H, s, $CH_2CON$), 8.12 and 8.4 (together 1H, s, CHO); $\nu_{max}$(KBr) 3350, 1760, 1680, 1600 $cm^{-1}$.

(b) Diphenylmethyl 7β-amino-3[(1-carbamoylmethyl-1H-tetrazol-5-yl)thiomethyl]-7α-formamidoceph-3-em-4-carboxylate.

7β-Amino-3-[(1-carbamoylmethyl-1*H*-tetrazol-5-yl) thiomethyl]-7α-formamidoceph-3-em-4-carboxylic acid, sodium salt was dissolved in water (2ml) and the pH adjusted to 2 by the addition of 5N hydrochloric acid. The precipitated solid was filtered off, washed with water and acetone, and dried *in vacuo* (75mg). The solid was suspended in acetonitrile (8ml) and excess diphenyldiazomethane added. After 12 days, the mixture

was treated with acetic acid (5 drops) for 1h. The mixture was poured into ethyl acetate-water and the organic layer separated, washed with saturated aqueous sodium hydrogen carbonate solution, brine, dried and evaporated. The residue was chromatographed on silica gel 60 (<230 mesh ASTM) to give the title compound (71mg) (50%); $\nu_{max}$ (Nujol) 3400br, 3280, 3250, 3200, 1750, 1720, 1710, 1690, 1675 $cm^{-1}$; $\delta(CD_3)_2CO$ $N\underline{H}_2$ obscured by solvent signal, 3.61 and 3.69 (2H, ABq, *J* 17.1 Hz, ring $S-CH_2$), 4.22 and 4.47 (2H, ABq, *J* 13.3 Hz), 5.13 (2H, s), 5.22 (1H, s), 6.89 (1H, broad s, exch. $D_2O$), 6.90 and 6.93 (together 1H, $\underline{CH}Ph_2$) 7.18-7.69 (11H, m, 1H exch $D_2O$), 7.92 and 8.08 (together 1H, d, *J* 12 Hz, and s, minor and major rotamers, $\underline{NH}$, exch. $D_2O$), 8.25 and 8.52 (together 1H, s and d *J* 12 Hz; major and minor rotamers respectively, latter signal collapses to s on $D_2O$ exch.).

Example 12

(a)
7β-Amino-3-[(1-(2-dimethylaminoethyl)-1H-tetrazol-5-yl)thiomethyl]-7α-formamidoceph-3-em-4-carboxylic acid sodium salt.

7β-Amino-7α-formamidocephalosporanic acid, trifluroacetic acid salt (320mg, 0.74mmol) in water (11ml) and acetone (3.5ml) was treated with saturated sodium hydrogen carbonate solution to pH 6.5. 1-(2-Dimethylaminoethyl)-1*H*-tetrazol-5-thiol (258mg, 1.49mmol) was added and the pH adjusted to 3.5. The solution was heated at 60°C under argon for 6h, cooled,

and the pH adjusted to 6.5. The solvents were evaporated and the residue chromatographed on Diaion HP20SS eluting with 2% acetone-water. The relevant fractions were combined, partially evaporated to remove acetone, and lyophilized to give the title compound (200mg, 60%); δ($D_2O$) 3.20 (6H, s, $N(CH_3)_2$), 3.02, and 3.77 (2H, ABq, J 17.6Hz, 2-$H_2$), 3.82 (2H, t, J 5.6Hz, N.$CH_2$), 4.05 and 4.22 (2H, ABq, J 13.5Hz, $CH_2$STet), 4.86(2H, t, J5.6Hz, N- $CH_2$), 5.08 and 5.17 (together 1H, s, C-6), 8.15 and 8.45 (together 1H, s, CHO); $\nu_{max}$ (KBr) 3360, 1760, 1670, 1600$cm^{-1}$.

(b) Diphenylmethyl 7β-amino-3[(1-dimethylaminoethyl -1H-tetrazol-5-yl)thiomethyl]-7α-formamidoceph-3 -em-4-carboxylate.

7β-Amino-3[(1-dimethylaminoethyl-1H-tetrazol-5-yl) thiomethyl]-7α-formamidoceph-3-em-4-carboxylic acid, sodium salt (60mg) was dissolved in water (2ml) and the pH adjusted to 2. The solution was evaporated, the residue dried *in vacuo*, suspended in acetonitrile (5ml), and treated with excess diphenyldiazomethane. After 2.1/4h. the mixture was poured into ethyl acetate-water and the organic layer separated, washed with saturated aqueous sodium hydrogen carbonate solution, brine, dried and evaporated. The residue was chromatographed on silica gel 60 (<230 mesh ASTM) to provide the title compound (50mg; 63%); $\nu_{max}$($CHCl_3$) 3400br, 1780, 1720sh, 1695$cm^{-1}$; δ($CDCl_3$) 2.23 (6H, s, $N(CH_3)_2$), 2.43 (2H, broad s, $NH_2$, exch. $D_2O$), 2.7 (2H, t, J 7Hz, $CH_2$N-) 3.62 (2H, s, ring S$CH_2$), 4.17-4.37 (3H, m, N-$CH_2$ + 1H of S-$CH_2$), 4.43 (1H, d, part of ABq,

J 14Hz, 1H of SCH$_2$), 5.16 (1H, s, 6-H), 6.61 (1H, s, NH, exch. $D_2O$), 6.98 (1H, s, CHPh$_2$), 7.2-7.6 (10H, m), 8.23 (1H, s, CHO). The product was contaminated with 20% of diphenylmethyl 7β-amino-3[(1-dimethylaminoethyl

-1$\underline{H}$-tetrazol-5-yl)thiomethyl]-7α-formamidoceph-2-em-4-carboxylate.

Example 13

3-[1-carbamoylmethyl-1H-tetrazol-5-yl)thiomethyl]-7β-[2-[(4-ethyl-2,3-dioxopiperazin-1-yl)-carbonylamino]-2-phenyl-acetamido]-7α-formamidoceph-3-em-4-carboxylic acid, sodium salt

7β-[D-2-[(4-Ethyl-2,3-dioxopiperazin-1-yl)carbonylamino-2-phenylacetamido]-7α-formamidocephalosporanic acid, sodium salt (0.070g, 0.104mmol) was dissolved in water (6ml) and acetone (2ml) and 5 mercapto-1$\underline{H}$-tetrazole-1-acetamide (0.033g, 0.208mmol) added. The pH of the resulting solution was adjusted to 3.5 and the resulting solution heated at 60$^{o}$C under an argon atmosphere for 11.5h. The resulting solution was concentrated and the concentrate taken up in a little saturated sodium hydrogen carbonate solution and then chromatographed on Diaion HP20SS. The fractions containing the title compound were freeze-dried to give the desired compound as a white amorphous solid (0.010g) $\nu_{max}$(KBr) 3280, 2970, 1775, 1710, 1670, and 1620 $cm^{-1}$, δ($D_2O$) 1.19 (3H, t, $\underline{J}$7Hz, N.$CH_2C\underline{H}_3$), 3.05 (2H, ABq, $\underline{J}$ 16Hz, 2-$CH_2$), 3.52 (2H, q, $\underline{J}$ 7Hz, N.$C\underline{H}_2$.$CH_3$), 3.64-3.76 and 3.94-4.07 (each 2H, m, diketopiperazine H), 4.25(2H, ABq, $\underline{J}$ 14 Hz, S.$CH_2$), 5.24 (1H, s, 6-H), 5.28 (2H, s, N.$CH_2$.CO), 5.52 (1H, s, Ph.C$\underline{H}$.NH), 7.37-7.59 (5H, m, aromatic H), and 8.13 and 8.44 (together 1H, each s, NH.C$\underline{H}$O).

MIC (μg/ml) against P. mirabilis 889 is 0.12

## Example 14

Sodium 3-[(1-carbamoylmethyl-1H-tetrazol-5-yl) thiomethyl]-7β-[D-2-(3,4-diacetoxyphenyl)-2-[(4-ethyl-2,3-dioxopiperazin-1-yl)carbonylamino]acetamido]-7α-formamidoceph-3-em-4-carboxylate.

(a) Diphenylmethyl 3-[(1-carbamoylmethyl-1H-tetrazol-5-yl)thiomethyl]-7β-[D-2-(3,4-diacetoxyphenyl)-2-[(4-ethyl-2,3-dioxopiperazin-1-yl)carbonylamino]acetamido]-7α-formamidoceph-3-em-4-carboxylate.

D-2-(3,4-Diacetoxyphenyl)-2-[(4-ethyl-2,3-dioxopiperazin-1-yl)carbonylamino]acetic acid (69mg; 0.158mmol) in dry dichloromethane (3ml) at 0°C containing a catalytic amount of dimethylformamide was cooled to 0°C and treated with oxalyl chloride (0.028ml). After 5 minutes, the cooling bath was removed, and the solution kept at room temperature for a further 45 minutes. The solvent was evaporated off and the residue dried *in vacuo*. Diphenylmethyl 7β-amino-3-[(1-carbamoylmethyl-1H-tetrazol-5-yl) thiomethyl]-7α-formamidoceph-3-em-4-carboxylate (62mg; 0.107mmol) and the crude acid chloride were dissolved in dry tetrahydrofuran (5ml) at -10°C and pyridine (9mg; 0.113mmole) in tetrahydrofuran (1ml) added. After 5 minutes the mixture was poured into ethyl acetate-water and the organic layer separated. The latter was washed successively with 0.1N hydrochloric acid, water, dilute aqueous sodium hydrogen carbonate

and water, dried and evaporated to give the title compound (110mg), contaminated with traces of starting material.

(b) Sodium 3-[(1-carbamoylmethyl-1H-tetrazol-5-yl) thiomethyl]-7β-[D-2-(3,4-diacetoxyphenyl)-2-[(4-ethyl-2,3-dioxopiperazin-1-yl)carbonylamino]acetamido]-7α-formamidoceph-3-em-4-carboxylate.

Diphenylmethyl 3-[(1-carbamoylmethyl-1H-tetrazol-5-yl) thiomethyl]-7β-[D-2-(3,4-diacetoxyphenyl)-2-[(4-ethyl-2,3-dioxopiperazin-1-yl)carbonylamino]acetamido]-7α-formamidoceph-3-em-4-carboxylate (110mg; containing some of corresponding 7β-amino compound) was dissolved in trifluoracetic acid. After 15 minutes, the solvent was evaporated off the residue dried *in vacuo*. The residual solid was suspended in water and the pH adjusted to 6.5 with dilute sodium hydrogen carbonate solution. The solution was chromatographed on HP20SS using acetone-water mixtures. The relevant fractions were combined, partially evaporated to remove acetone, and then freeze-dried to give the title compound (37mg). $\nu_{max}$(KBr), 3300br, 1775, 1680br, 1625cm$^{-1}$; δ($D_2O$) *inter alia* 1.19 (3H, t, *J* 7.2Hz), 2.33 (6H,s, 2x$OCOCH_3$), 2.8 and 3.18 (2H, ABq, *J* 16.5Hz, S-C*H*$_2$), 3.51 (2H, q, J 7.2Hz), 3.68 (2H, m) 4.0 (2H, m), 4.08 and 4.48 (2H, ABq, *J* 14.6Hz, -$CH_2$-S), 5.22 (1H, s, 6-H), 5.28 (2H, AA', $CH_2$CON), 5.54 (1H, s, Ar CH), 7.27-7.58 (3H, m, aromatics), 8.13 (1H, s, C*H*O).

MIC against *P.mirabilis* is 0.12μg/ml.

Example 15

3-[(1-Carboxymethyl-1H-tetrazol-5-yl)thiomethyl]-7β-[D-2(3,4-dihydroxyphenyl)-2-[(4-ethyl-2,3-dioxopiperazin-1-yl)carbonylamino]acetamido]-7α-formamidoceph-3-em-4-carboxylic acid, disodium salt

To a solution of 3-[(1-carboxymethyl-1*H*-tetrazol-5-yl)thiomethyl]-7β-[D-2(3,4-diacetoxyphenyl)-2-[(4-ethyl-2,3-dioxopiperazin-1-yl)carbonylamino]acetamido]-7α-formamidoceph-3-em-4-carboxylic acid, disodium salt (48mg) in tetrahydrofuran (2ml) and water (3ml) at pH 9.0 was added sodium sulphite (17.7mg) and the solution stirred. The pH was maintained at 9.0 and after 70 min more sodium sulphite (2mg) was added. After a total of 2h the pH was adjusted to 6.7, the solution concentrated and then chromatographed on Diaion HP20SS. The relevant fractions were lyophilised to give the title compound (23mg, 53%) as an amorphous white solid; $\nu_{max.}$(KBr) 3430, 2980, 1765, 1675, 1625, 1524, and 1460cm$^{-1}$, δ($D_2O$) *inter alia* 1.18 (3H, t, *J* 7Hz, $N.CH_2.CH_3$), [(3.01 and 3.40) and (3.05 and 3.45) together 2H, each ABq, *J* 17Hz, 2-$CH_2$, major and minor rotamer respectively], 3.50 (2H, q, *J*7Hz, $N.CH_2.CH_3$), 3.6-3.8(2H, m, $N.CH_2.CH_2.N$), 3.9-4.15 (3H, m, $N.CH_2.CH_2.N$ and high-field half of 3-$CH_2S$), 4.33 and 4.39 [together 1H, low-field half of 3-$CH_2S$ ABq (*J*14Hz)], 5.00 (2H,s, $N.CH_2.CO_2$), 5.18 and 5.23 (together 1H, each s, 6-H, minor and major rotamer respectively), 5.26 and 5.31 (together 1H, each s, NH.CH.CO, minor and major rotamer respectively), 6.8-7.1 (3H,m, aromatics), 8.10 and 8.41 (together 1H, each s, NH.CHO, major and minor rotamer respectively).

MIC (μg/ml) against *P.Mirablis* 889 is 0.12.

Example 16

3[(6-Carbamoyl-2-methyl-5-oxo-4H-1,2,4-triazin-3-yl)thiomethyl]-7β-[D-2[(4-ethyl-2,3-dioxopiperazin-1-yl)carbonylamino]-2-phenylacetamido]-7α-formamidoceph-3-em-4-carboxylic acid, sodium salt

(a) Diphenylmethyl 7β-amino-3[(6-carbamoyl-2-methyl-5-oxo-4H-1,2,4-triazin-3-yl)thiomethyl]-7α-formamidoceph-3-em-4-carboxylate

7β-Amino-7α-formamidocephalosporanic acid, trifluoroacetic acid salt (482mg, 1.13mmol) in water (6ml) and acetone (3ml) was treated with sodium hydrogen carbonate solution to pH 6.0. 6-Carbamoyl-2-methyl-5-oxo-4H-1,2,4-triazin-3-thiol (186mg, 1mmol) was added, pH adjusted to 5.4 with 2N-hydrochloric acid and the solution heated under argon at 60°C. After 2h 45min, the pH was adjusted to 6.5, mixture filtered and solvent evaporated. The residue was chromatographed on Diaion HP20SS eluting with water-acetone mixture. Lyophilization of the relevant fraction gave 7β-amino-3[(6-carbamoyl-2-methyl-5-oxo-4H-1,2,4-triazin-3-yl)thiomethyl]-7α-formamidoceph-3-em-4-carboxylic acid, sodium salt (120mg) as an off-white solid, $\nu_{max.}$ (Nujol) 3150, 3000, 1760, 1700, 1690, 1680, 1610, and 1550cm$^{-1}$.

The above mentioned salt was dissolved in water and pH adjusted to 3.0. The resulting solution was

lyophilised to give an amorphous solid which was suspended in acetonitrile (5ml) and treated with excess diphenyldiazomethane. After 7 days, the mixture was treated with acetic acid (5 drops) for 1h. The mixture was poured into ethyl acetate-water and the organic layer separated, washed with saturated aqueous sodium hydrogen carbonate, brine, dried ($MgSO_4$) and evaporated. The residue was chromatographed on silica gel 60 (<230 mesh ASTM) to give the title compound (13mg) as a white solid; $\nu_{max.}$($CHCl_3$) 3470, 3410, 3300, 3000, 1780, 1710, 1705, 1700, 1650 and 1555 $cm^{-1}$, $\delta$[$(CD_3)_2CO$] 3.62 and 3.76 (2H, ABq, J17Hz, 2-$CH_2$), 3.85 (3H,s, N.$CH_3$), 4.14 and 4.54 (2H, ABq, J14Hz, 3-$CH_2$S), 5.11 and 5.20(together 1H, each s, 6-H, minor and major rotamer respectively), 6.99 (1H,s, CH.$Ph_2$), 7.2-7.7 (12H, m, aromatics and 2xamide protons), 8.25 and 8.54 (together 1H, each s, NH.CHO, major and minor rotamer respectively), 8.99 br (1H, amide proton). (Addition of $D_2O$ caused the signal at 8.99 to disappear whilst that at 7.2-7.7 reduced in intensity).

(b) Diphenylmethyl 3[(6-carbamoyl-2-methyl-5-oxo-4H-1,2,4-triazin-3-yl)thiomethyl]-7β-[D-2[(4-ethyl-2,3-dioxopiperazin-1-yl)carbonylamino]-2-phenylacetamido] -7α-formamidoceph-3-em-4-carboxylate

Freshly prepared D-2[(4-ethyl-2,3-dioxopiperazin-1-yl)carbonylamino]-2-phenylacetyl chloride, generated by the treatment of the corresponding acid (20.4mg, 0.064mmol) with oxalyl chloride (16.3mg, 0.128mmol) in dry dichloromethane (2ml) containing N,N-dimethylformamide ($^1/_{10}$ drop) for

2h, was taken up in dry dichloromethane (2ml) and added to a stirred solution of diphenylmethyl 7β-amino-3[(6-carbamoyl-2-methyl-5-oxo-4H-1,2,4-triazin-3-yl) thiomethyl]-7α-formamidoceph-3-em-4-carboxylate (26mg, 0.0427mmol) in dichloromethane (1ml), followed by pyridine (3.4mg, 0.0427mmol). After 90 min, the mixture was washed successively with dilute aqueous sodium hydrogen carbonate, dilute hydrochloric acid, and saturated brine, dried ($MgSO_4$) and evaporated. The residue was chromatographed on silica gel 60 to give the title compound (19mg) as a white solid; δ[$(CD_3)_2CO$] 1.15 (3H, t, J7Hz,N.$CH_2$.$CH_3$), 3.22 and 3.43 (2H, ABq, J16Hz, 2-$CH_2$), 3.47 (2H, q, J7Hz, N.$CH_2$.$CH_3$), 3.6-3.8 (2H,m,N.$CH_2$.$CH_2$.N), 3.82 and 3.84 (together 3H, each s, N.$CH_3$, major and minor rotamer respectively), 3.95-4.10 (2H,m,N.$CH_2$.$CH_2$.N), 4.17 and 4.53 (2H,ABq,J13.5Hz, 3-$CH_2$S), 5.27 and 5.30 (together 1H, each s, 6-H, major and minor rotamer respectively), 5.70 (1H,d,J7.5Hz, NH.CH.CO), 6.96 (1H,s,CH.$Ph_2$), 7.2-7.8 (17H,m, aromatics and 2 amide protons), 8.02 (1H,s,CO.NH.C), 8.27 and 8.53 (together 1H, each s, NH.CHO, major and minor rotamer respectively), 8.63 and 8.88 br (together 1H, NHCHO, minor and major rotamer respectively).

(c)

<u>3[(6-Carbamoyl-2-methyl-5-oxo-4H-1,2,4-triazin-3-yl) thiomethyl]-7β-[D-2[4-ethyl-2,3-dioxopiperazin-1-yl) carbonylamino]-2-phenylacetamido]-7α-formamidoceph-3-em -4-carboxylic acid, sodium salt.</u>

Diphenylmethyl 3[(6-carbamoyl-2-methyl-5-oxo-4H-1,2,4-triazin-3-yl)thiomethyl]-7β-[D-2[(4-ethyl-2,3-

dioxopiperazin-1-yl)carbonylamino]-2-phenylacetamido]-7α-formamidoceph-3-em-4-carboxylate (19mg) was treated with trifluoroacetic acid (2ml) and allowed to stand at room temperature. After 10 min the solvent was evaporated and the residue taken up in saturated aqueous sodium hydrogen carbonate solution. The resulting cloudy suspension was chromatographed on Diaion HP20SS eluting with water-acetone mixture. Lyophilization of the relevant fractions gave the title compound (11mg) as a white amorphous solid; $\nu_{max}$. (KBr) 3390, 2990, 2365, 2360, 2325, 1770, 1680, 1555 and 1520 $cm^{-1}$, δ($D_2O$) 1.20 (3H,t,<u>J</u>7Hz,N.$CH_2$.C<u>H</u>$_3$), 3.11 and 3.15 (together 1H, each d, <u>J</u>17Hz, high-field half of 2-$CH_2$ ABq, major and minor rotamer respectively), 3.45 -3.60 (together 3H,m,N.C<u>H</u>$_2$.$CH_3$ and low-field half of 2-$CH_2$ ABq, q centered at 3.54 discerneable), 3.6-3.8 (2H,m,N.$CH_2$.C<u>H</u>$_2$.N), 3.95 and 3.97 (together 3H, each s, N.$CH_3$, major and minor rotamer respectively), 3.9-4.2 (together 3H,m,N.C<u>H</u>$_2$.$CH_2$.N and high-field half of 3-$CH_2$S), 4.37 and 4.41 (together 1H, each d, <u>J</u>14Hz, low-field half of 3-$CH_2$S ABq, major and minor rotamer respectively), 5.25 and 5.28 (together 1H, each s, 6-H, minor and major rotamer respectively), 5.47 and 5.52 (together 1H, each s, NH.C<u>H</u>.CO, minor and major rotamer respectively), 7.4-7.6 (5H,m, aromatics) 8.15 and 8.47 (together 1H, each s, NH.C<u>H</u>O, major and minor rotamer respectively).

MIC (μg/ml) against <u>P.Mirabilis</u> 889 is 0.25

<u>Example 17</u>

<u>3[(1-Dimethylaminoethyl-1H-tetrazol-5-yl)thiomethyl]</u>

-7β-[[2-D(4-ethyl-2,3-dioxopiperazin-1-yl)carbonylamino]-2-phenylacetamido]-7α-formamidoceph-3-em-4-carboxylic acid, sodium salt

(a) Diphenylmethyl 3[(1-dimethylaminoethyl-1H-tetrazol-5-yl)thiomethyl]-7β-[[2-D(4-ethyl-2,3-dioxopiperazin-1-yl)carbonylamino]-2-phenylacetamido]-7α-formamidoceph-3-em-4-carboxylate

Freshly prepared D-2[(4-ethyl-2,3-dioxopiperazin-1-yl)carbonylamino]-2-phenylacetyl chloride, generated by treatment of the corresponding acid (113mg, 0.354mmol) with oxalyl chloride (89.8mg, 0.707mmol) in dry dichloromethane (5ml) containing N,N-dimethylformamide ($^{1}/_{10}$ drop) for 1.25h, was taken up in dichloromethane (2.5ml) and added to a stirred solution of diphenylmethyl 7β-amino-3[(1-dimethylaminoethyl-1H-tetrazol-5-yl)thiomethyl]-7α-formamidoceph-3-em-4-carboxylate (140mg, 0.236mmol) containing about 50% of the corresponding delta-2 isomer, followed by pyridine (20.5mg, 0.259mmol). After 15 min, the mixture was diluted with dichloromethane and washed successively with dilute aqueous sodium hydrogen carbonate, dilute hydrochloric acid, and saturated brine, dried ($MgSO_4$) and evaporated. The residue was chromatographed on silica gel to give the title compound (125 mg), containing about 50% of the corresponding delta-2 isomer, as a pale glass; $\nu_{max}$.($CHCl_3$) 3300, 3000, 1780, 1720, 1700 and 1685$cm^{-1}$, δ($CDCl_3$/$D_2O$) inter alia 1.20 (t,N.$CH_2$.$CH_3$), 2.24 (2xs,$NMe_2$), 2.6-2.7(t,$CH_2$.$NMe_2$), 5.30 (s,6-H), 5.46 (s,NH.CH.CO), 6.89 (s, CH.$Ph_2$), 8.22 and 8.45 (each s, NH.CHO, major and minor rotamer

respectively).

(b) <u>3[(1-Dimethylaminoethyl-1H-tetrazol-5-yl)thiomethyl]-7β-[[2-D(4-ethyl-2,3-dioxopiperazin-1-yl)carbonylamino]-2-phenylacetamido]-7α-formamidoceph-3-em-4-carboxylic acid, sodium salt</u>

Diphenylethyl 3[(1-dimethylaminoethyl-1H-tetrazol-5-yl)thiomethyl]-7β-[[2-D(4-ethyl-2,3-dioxopiperazin-1-yl)carbonylamino]-2-phenylacetamido]-7α-formamidoceph-3-em-4-carboxylate, containing approximately 50% of the corresponding delta 2 isomer, (100mg) was taken up in trifluoroacetic acid (5ml) for 10 min and the solvent then evaporated *in vacuo*. The residue was layered with water, pH adjusted to 6.8 and the suspension resulting from vigorous shaking purified on Diaion HP20SS. Lyophilisation of the relevant fractions gave the title compound (32mg) containing approximately 50% of the corresponding delta 2 isomer as a white solid; $\nu_{max.}$(KBr) 3430, 2985, 1770, 1680, 1610, 1505, 1460, 1395, 1365, and 1185cm$^{-1}$, δ($D_2O$) *inter alia* 1.19 (t,N.$CH_2$.C<u>H</u>$_3$), 2.95 (s,$NMe_2$), 3.14 [high-field half of 2-$CH_2$ ABq (<u>J</u>17Hz)], 5.26(s,6-H), 5.50 (s, NH.C<u>H</u>.CO), 7.3-7.6 (m, aromatics), and 8.13 and 8.30 (each s, NH.C<u>H</u>O).

MIC (μg/ml) against <u>P.Mirabilis</u> 889 is 1.0

Claims

1. A compound of formula (I) or a pharmaceutically acceptable salt or in vivo hydrolysable ester thereof:

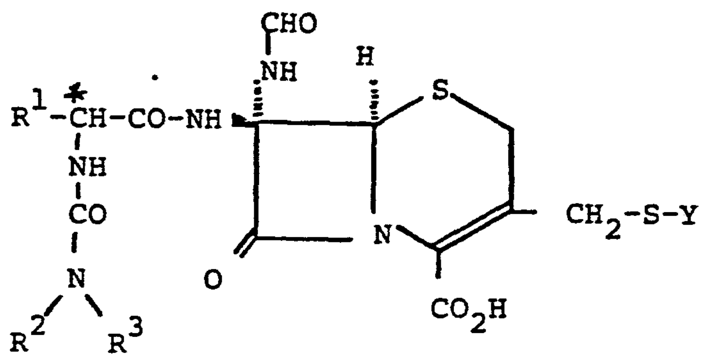

(I)

wherein Y is a group of formula (a), (b), (c), or (d):

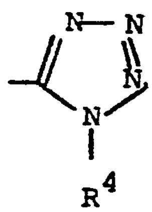

(a)

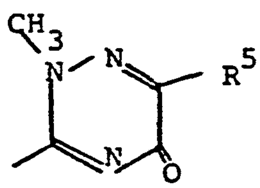

(c)

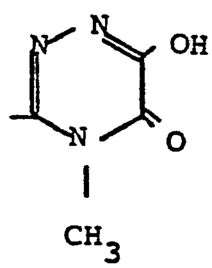

(b)

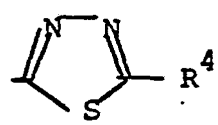

(d)

$R^1$ is phenyl, substituted phenyl, cyclohexenyl, cyclohexadienyl, or a 5- or 6-membered heterocyclic ring containing up to three hetero-atoms selected from oxygen, sulphur or nitrogen, optionally substituted with hydroxy, amino, halogen, substituted amino or $C_{1-6}$ alkoxy; $R^2$ is hydrogen or an optionally substituted $C_{1-6}$ alkyl group and $R^3$ is

an optionally substituted 5- or 6- membered heterocyclic group containing one or two nitrogen heteroatoms; or $R^2$ and $R^3$ together with the nitrogen atom to which they are attached form an optionally substituted five- or six-membered heterocyclic group containing one or two nitrogen heteroatoms;

$R^4$ is carbamoyl, carbamoyl ($C_{1-6}$) alkyl, amino ($C_{1-6}$) alkyl, di ($C_{1-6}$ alkyl) amino ($C_{1-6}$) alkyl, hydroxy ($C_{1-6}$) alkyl, sulpho ($C_{1-6}$) alkyl, $C_{2-6}$ alkenyl or carboxy ($C_{1-6}$) alkyl; and $R^5$ is carbamoyl or $C_{1-6}$ alkyl.

2. A compound as claimed in claim 1 wherein Y is

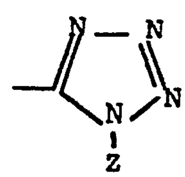

or

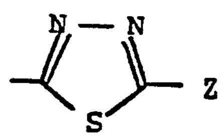

wherein Z is carbamoyl, carbamoyl ($C_{1-6}$) alkyl, amino ($C_{1-6}$) alkyl, di-($C_{1_6}$ alkyl) amino ($C_{1-6}$) alkyl, hydroxy ($C_{1-6}$) alkyl, sulpho ($C_{1-6}$) alkyl, or $C_{2-6}$ alkenyl.

3. A compound as claimed in claim 1 wherein Y is

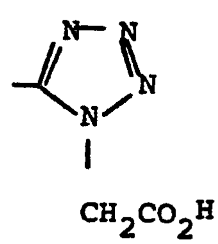

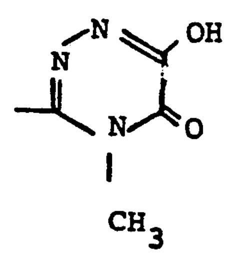

4. A compound as claimed in any one of claims 1 to 3 of formula (II) or a pharmaceutically acceptable salt

or in-vivo hydrolysable ester thereof:

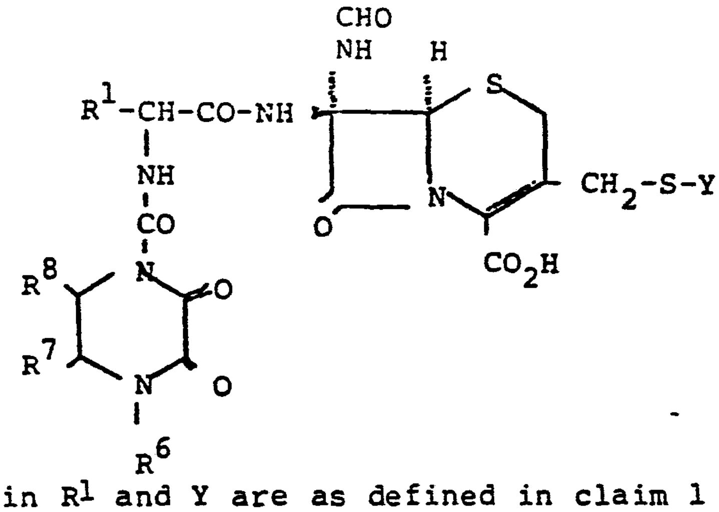

(II)

wherein $R^1$ and Y are as defined in claim 1 and $R^6$ represents hydrogen, $C_{1-6}$ alkyl, substituted alkyl, aryl, or aralkyl; $R^7$ and $R^8$ are the same or different and represent hydrogen, $C_{1-6}$ alkyl, substituted alkyl, halogen, amino, hydroxy or $C_{1-6}$ alkoxy or $R^7$ and $R^8$ form the residue of 5- or 6- membered carbocyclic or heterocyclic ring.

5. A compound as claimed in claim 1 selected from the following or a pharmaceutically acceptable salt or in vivo hydrolysable ester thereof:

a) 7β-[DL-2-(3,4-Diacetoxyphenyl)-2[(4-ethyl-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid;

3-[(1-Carboxymethyl-1H-tetrazol-5-yl)thiomethyl]-7β-[D-2-[(4-ethyl-2,3-dioxopiperazin-1-yl)carbonylamino]-2-phenylacetamido]-7α-formamidoceph-3-em-4-carboxylic acid;

b) 7β[D-2-[(4-Ethyl-2,3-dioxopiperazin-1-yl)carbonylamino]-2-phenylacetamido]-7α-formamido

-3-[(6-hydroxy-4-methyl-5-oxo-4H-1,2,4-triazin-3-yl)thiomethyl]ceph-3-em-4-carboxylic acid;

7β-[D-2(4-Acetoxyphenyl)-2-[(4-ethyl-2,3-dioxopiperazin -1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid;

7β-[DL-2-[(4-Ethyl-2,3-dioxopiperazin-1-yl)carbonylamino]-2-(thien-2-yl)acetamido]-7α-formamido-3-[(6-hydroxy-4-methyl-5-oxo-4H-1,2,4-triazin-3-yl)thiomethyl]ceph-3-em-4-carboxylic acid;

7β-[DL-2[(4-Ethyl-2,3-dioxopiperazin-1-yl)carbonylamino]-2-(2-fluorophenyl)acetamido]-7α-formamido-3-[(6-hydroxy-4-methyl-5-oxo-4H-1,2,4-triazin-3-yl)thiomethyl]ceph-3-em-4-carboxylic acid;

7β-[D-2(3,4-Diacetoxyphenyl)-2-[(4-ethyl-2,3-dioxopiperazin-1-yl)carbonylamino]acetamido]-7α-formamido-3-[(6-hydroxy-4-methyl-5-oxo-4H-1,2,4-triazin-3-yl)thiomethyl]ceph-3-em-4-carboxylic acid.

3-[(1-Carboxymethyl-1H-tetrazol-5-yl)thiomethyl]-7β-[D-2(3,4-diacetoxyphenyl)-2-[(4-ethyl-2,3-dioxopiperazin-1-yl)carbonylamino]acetamido]-7α-formamidoceph-3-em-4-carboxylic acid;

c) 3-(1-carbamoylmethyl-1H-tetrazol-5-yl)thiomethyl]-7α-[D-2-[(4-ethyl-2,3-dioxopiperazin-1-yl)-

carbonylamino[-2-phenylacetamido]-7α-formamidoceph-3-em-4-carboxylic acid;

d) 3-[(1-Carbamoylmethyl-1H-tetrazol-5-yl)thiomethyl]-7β-[D-2-(3,4-diacetoxyphenyl)-2-[(4-ethyl-2,3-dioxopiperazin-1-yl)carbonylamino]-7α-formamidoceph-3-em-4-carboxylic acid;

e) 3-[(1-Carboxymethyl-1H-tetrazol-5-yl)thiomethyl]-7β-[D-2(3,4-dihydroxyphenyl)-2-[(4-ethyl-2,3-dioxopiperazin-1-yl)carbonylamino]acetamido]-7α-formamidoceph-3-em-4-carboxylic acid;

3-[(1-Dimethylaminoethyl-1H-tetrazol-5-yl)thiomethyl]-7β-[(2-D(4-ethyl-2,3-dioxopiperazin-1-yl)carbonylamino]-2-phenylacetamido]-7α-formamidoceph-3-em-4-carboxylic acid; or

f) 3-[(6-Carbamoyl-2-methyl-5-oxo-4H-1,2,4-triazin-3-yl)thiomethyl]-7β-[D-2[(4-ethyl-2,3-dioxopiperazin-1-yl)carbonylamino]-2-phenylacetamido]-7α-formamidoceph-3-em-4-carboxylic acid.

6. A pharmaceutical composition comprising a compound as claimed in claim 1 together with a pharmaceutically acceptable carrier or excipient.

7. A pharmaceutical composition as claimed in claim 6 which further comprises a β-lactamase inhibitor.

8. A compound as claimed in claim 1 for use in the treatment of bacterial infections.

9. A process for the preparation of a compound as

claimed in claim 1 which process comprises:

a) formylating a compound of formula (V)

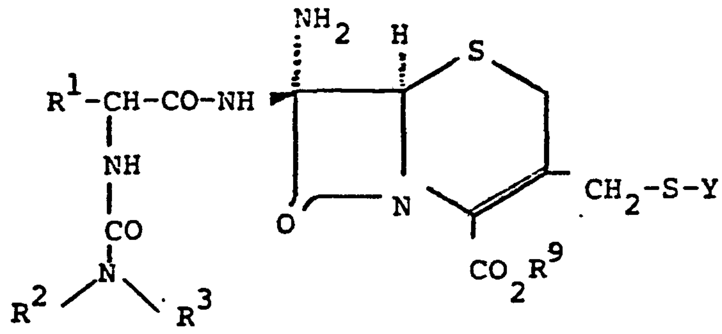

(V)

wherein $R^1$, $R^2$, $R^3$, and Y are in hereinbefore defined and wherein any reactive groups may be protected; and $R^9$ is a readily removable carboxy protecting group; or

b) reacting a compound of formula (VI):

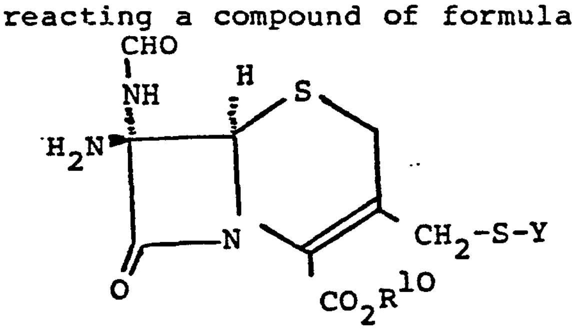

(VI)

wherein Y is as hereinbefore defined and wherein any reactive groups may be protected; the amino group is optionally substituted with a group which permits acylation to take place and $R^{10}$ is hydrogen or a group $R^9$ as hereinbefore defined with an N-acylating derivative of an acid of formula (VII):

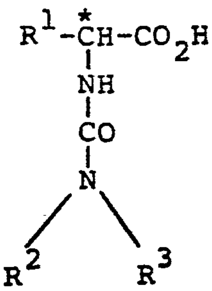

(VII)

wherein $R^1$, $R^2$ and $R^3$ are as defined with respect to formula (I) and wherein any reactive groups therein may be protected; or

c) reacting a compound of formula (VIII):

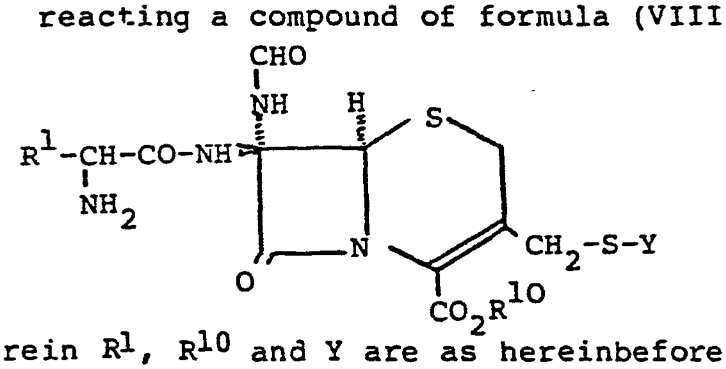

(VIII)

wherein $R^1$, $R^{10}$ and Y are as hereinbefore defined and the α-amino group is optionally substituted with a group which permits acylation to take place, and any reactive groups may be protected; with an N-acylating derivative of an acid of formula (IX):

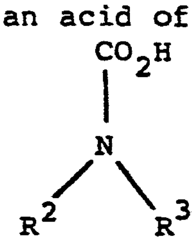

(IX)

wherein $R^2$ and $R^3$ are as hereinbefore defined and wherein any reactive groups may be protected;

or d) reacting a compound of formula (XIII):

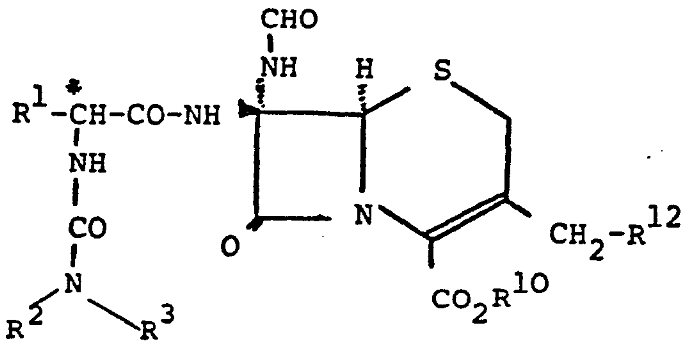

(XIII)

wherein $R^1$, $R^2$, $R^3$ and $R^9$ are as defined hereinbefore and wherein any reactive groups may be protected and $R^2$ is a leaving group; with a thiol of formula:

Y-SH

with the proviso that when $R^{12}$ is an acyloxy group $-CO_2R^{10}$ must be in the free acid form or a salt thereof; and after any of processes a), b), c) or d), if necessary, carrying out one or more of the following steps:

i) removing any carboxyl-protecting group $R^9$;
ii) removing any protecting groups on $R^1$, $R^2$, $R^3$, or Y;
iii) converting the product into a salt or <u>in vivo</u> hydrolysable ester thereof.

10. A compound selected from the following or a salt thereof:

7β-Amino-3-[(1-carboxymethyl-1<u>H</u>-tetrazol-5-yl) thiomethyl]-7α-formamidoceph-3-em-4-carboxylic acid bis-diphenylmethyl ester

7β-Amino-3-[(1-carbamoylmethyl-1-<u>H</u>-tetrazol-5-yl)

thiomethyl]-7α-formamidoceph-3-em-4-carboxylic acid or the diphenylmethyl ester thereof;

7β-Amino-3-[(1-(2-dimethylaminoethyl)-1*H*-tetrazol-5-yl)thiomethyl]-7α-formamidoceph-3-em-4-carboxylic acid or the diphenylmethyl ester thereof;

and 7β-amino-3[(6-carbamoyl-2-methyl-5-oxo-4*H*-1,2,4-triazin-3-yl)thiomethyl]-7α-formamidoceph-3-em-4-carboxylate or the diphenylmethyl ester thereof.